# FASCICULE DE BREVET EUROPEEN

(11) **EP 2 157 983 B1**
(45) Date de publication et mention de la délivrance du brevet: **10.01.2018**
(21) Numéro de dépôt: 08826154.0
(22) Date de dépôt: 10.06.2008
(51) Int. Cl.: A61L 27/24, A61L 27/56, A61L 31/04, A61L 31/16

(54) **SUPPORT COLLAGENIQUE MODIFIE PAR GREFFAGE COVALENT DE MOLECULES D'ADHESION**
MITTELS KOVALENZBINDUNG VON ADHÄSIONSMOLEKÜLEN MODIFIZIERTES KOLLAGENGERÜST
COLLAGEN SCAFFOLD MODIFIED BY COVALENT GRAFTING OF ADHESION MOLECULES

(30) Priorité: 13.06.2007 FR 0704209
(43) Date de publication de la demande: 03.03.2010
(73) Titulaire: Schussler, Olivier, 92150 Suresnes (FR)
(72) Inventeur: Schussler, Olivier, 92150 Suresnes (FR)
(74) Mandataire: Jacobacci Coralis Harle
(86) Numéro de dépôt international: PCT/FR2008/000785
(87) Numéro de publication internationale: WO 2009/007531

(56) Documents cités:
- WO-A-2006/037770
- US-A1- 2005 123 582
- US-B1- 6 608 040
- MYLES J L ET AL: "Modification of the adhesive properties of collagen by covalent grafting with RGD peptides." JOURNAL OF BIOMATERIALS SCIENCE. POLYMER EDITION 2000, vol. 11, no. 1, 2000, pages 69-86, XP009098120 ISSN: 0920-5063 cité dans la demande
- CHANG Y ET AL: "Reconstruction of the right ventricular outflow tract with a bovine jugular vein graft fixed with a naturally occurring crosslinking agent (genipin) in a canine model." THE JOURNAL OF THORACIC AND CARDIOVASCULAR SURGERY DEC 2001, vol. 122, no. 6, décembre 2001 (2001-12), pages 1208-1218, XP002475075 ISSN: 0022-5223
- SCHUSSLER OLIVIER ET AL: "RGD-coupling to collagen scaffold improves cardiomyocyte viability and contractility: New possibilities for cardiac tissue engineering" CIRCULATION, vol. 116, no. 16, Suppl. S, octobre 2007 (2007-10), page 70, XP002475076 & 80TH ANNUAL SCIENTIFIC SESSION OF THE AMERICAN-HEART-ASSOCIATION; ORLANDO, FL, USA; NOVEMBER 04 -07, 2007 ISSN: 0009-7322
- M. RADISIC ET AL: "From the Cover: Functional assembly of engineered myocardium by electrical stimulation of cardiac myocytes cultured on scaffolds", PROCEEDINGS OF THE NATIONAL ACADEMY OF SCIENCES, vol. 101, no. 52, 28 December 2004 (2004-12-28), pages 18129-18134, XP055083856, ISSN: 0027-8424, DOI: 10.1073/pnas.0407817101
- STASKOWSKI ET AL: "The histologic fate of autologous collagen injected into the canine vocal fold", OTOLARYNGOLOGY AND HEAD AND NECK SURGERY, ROCHESTER, US, vol. 118, no. 2, 1 February 1998 (1998-02-01), pages 187-190, XP005508356, ISSN: 0194-5998, DOI: 10.1016/S0194-5998(98)80010-7

## Description

La présente invention a trait à des supports collagéniques caractérisés en ce qu'ils sont modifiés chimiquement par greffage covalent de molécules d'adhésion qui facilitent l'implantation de cellules, leur survie et leur différenciation. Le support collagénique (désigné ci-après par support ou échafaudage ; « scaffold »en anglais) est utilisé avec des cellules à potentiel contractile mais d'autres types cellulaires comme des cellules à potentiel angiogénique peuvent aussi être associés.

L'invention porte également sur le procédé de fabrication dans le domaine médecine/chirurgie d'un support collagénique tridimensionnel destiné à la thérapie cellulaire et/ou à l'ingénierie de tissu contractile et l'utilisation du support collagénique tridimensionnel dans la réalisation de différents dispositifs du domaine médecine/chirurgie cardio-thoracique et vasculaire.

La présente invention concerne des substrats collagéniques revêtus, en particulier à usage médical, pour la réparation et la régénération tissulaire.

Le collagène est un constituant de nombreux dispositifs utilisés en thérapie cellulaire, pour l'ingénierie tissulaire et dans le domaine de la médecine/chirurgie cardio-thoracique et vasculaire. Ces dispositifs sont destinés à être implantés et cellularisés et leur fonctionnalité est optimisée par une meilleure interaction entre les cellules et les supports.

Le collagène est amélioré par greffage covalent de molécules d'adhésion qui peuvent ainsi interagir de manière optimale avec les récepteurs membranaires de cellules d'intérêt (cellules associées ou qui vont coloniser le support). L'activation des récepteurs entraîne une cascade d'événements qui favorisent l'adhésion, la croissance, la survie, contrôlent l'apoptose et la différenciation cellulaire.

L'invention propose également des méthodes plus générales : 1) pour améliorer la fixation de molécules comme des molécules d'adhésion ou agents groupes carboxyles comme c'est le cas pour le collagène, 2) pour améliorer la biocompatibilité de l'échafaudage de collagène par une réticulation mettant en oeuvre des réactions chimiques ne conduisant pas à des produits toxiques susceptibles de gêner la croissance in situ des cellules.

Actuellement les techniques de régénération tissulaire font appel à différentes techniques : a) la transplantation directe de cellules libres dans les environnements endommagés b) des techniques d'ingénierie tissulaire avec développement de tissu de remplacement à partir de cellules mises en place dans des échafaudages tridimensionnels. La réalisation d'échafaudages pour la thérapie cellulaire est difficile car il faut que ce support permette la libre diffusion des nutriments et de l'oxygène, possède les propriétés mécaniques mais également les ligands biologiques spécifiques lui permettant d'interagir avec les cellules d'intérêt associées afin de favoriser en outre leur survie et leur différenciation. De plus, ce support doit favoriser l'angiogénèse car il n'est pas vascularisé, contrairement aux tissus naturels. Ce support doit être biocompatible et n'entraîner que peu de réaction inflammatoire après implantation in vivo. Enfin, il faut que ce support puisse être utilisé en clinique, qu'il soit compatible avec la sécurité sanitaire, ce qui exclut l'emploi d'un extrait tumoral. Jusqu'à maintenant de tels supports n'existent pas pour l'ingénierie d'un tissu cardiovasculaire et thoracique.

Jusqu'à présent les techniques de transplantation de cellules isolées ont montré leurs limites avec une mortalité cellulaire très importante et souvent l'absence de différenciation des cellules transplantées. On connaît ainsi de T. Yasuada et al.¹ que l'emploi de cardiomyocytes foetaux, de myoblastes squelettiques et de cellules souches de moelle a toujours rencontré un succès limité dans la capacité à reconstituer des tissus endommagés et dans l'amélioration de la fonction cardiaque. Le manque de production de nouvelles fibres myocardiques en nombre suffisant pour qu'un effet positif ait une traduction clinique a été attribué à la mort très importante des cellules se produisant après la greffe (autour de 90%) et à l'incapacité de la cellule greffée à se différencier et à s'intégrer dans le myocarde^{1,2}. Le faible taux de survie des cellules transplantées a été souligné dans plusieurs travaux comme pouvant expliquer le manque de succès des thérapies cellulaire où les cellules sont injectées isolément. Ce problème de survie a été illustré par plusieurs groupes qui ont montré que la modification de cellules avec des gènes de survie pouvait considérablement améliorer leur survie et leur fonctionnalité après transplantation³.

Plus récemment encore, différents groupes ont montré l'intérêt d'associer les cellules à un support tridimensionnel qui permettrait de recréer l'environnement tissulaire natif avec la transplantation de cellules dans des supports synthétiques ou biologiques comme par exemple le collagène ⁴⁻⁸. La destruction de la matrice extracellulaire (ECM) native principalement composée de collagène de type I et III dans le myocarde et son remplacement par un tissu aux propriétés mécaniques altérées (moins compliant), moins vascularisé et qui, de surplus, du fait d'une modification de sa composition, ne permet plus une interaction optimale pourrait expliquer les résultats assez médiocres de la transplantation de cellules libres. La présence d'une matrice collagénique améliore ainsi significativement la régénération du tissus musculaire⁹. L'injection de cellules par voie endovasculaire est en cours d'évaluation pour la régénération myocardique et pourrait avoir un certain intérêt ¹⁰. Des injections de microsphères de collagène ont également été réalisées par voie vasculaire portale avant d'injecter des cellules de type hépatocyte dans les mêmes vaisseaux ou dans les territoires vascularisés ou de drainage de ces vaisseaux afin de favoriser la survie ¹¹. Des gels ou hydrogels injectables de collagène peuvent également être obtenus en associant des microparticules de collagène avec des particules synthétiques comme des nanotubes de carbone ¹² ou à des polymères naturels comme le chitosane.

L'identification des caractéristiques du microenvironnement qui affecte le phénotype cellulaire et sa fonction dans une matrice de collagène fournit les conditions pour l'ingénierie de tissu myocardique artificiel en vue d'applications médicales^{13,14}. Dans les tissus, cet environnement 3D est constitué par la matrice extracellulaire qui est constituée de protéines. L'une des principales protéines structurales de cet environnement est le collagène ¹⁵. Les cellules interagissent avec cet environnement par le biais de récepteurs de surface appelés « intégrines » qui reconnaissent des séquences élémentaires le plus souvent peptidiques et présentes sur les protéines de l'ECM ^{13,16}. Il s'agit de récepteurs hétérodimériques transmembranaires formés par l'association d'une chaîne alpha et d'une chaîne bêta. L'activation de ces récepteurs va dépendre de la présence du ligand sur l'ECM mais également de la présentation de ce ligand et des possibilités de regrouper les différents récepteurs. L'activation du récepteur de la molécule ou de la séquence peptidique d'adhésion, le récepteur intégrinique, est responsable d'une cascade d'événements intracellulaires avec remaniements du cytosquelette de la cellule, activation de différentes voies de signalisation qui contrôlent la survie^{17,18}, la forme ¹⁹, la prolifération, l'apoptose¹⁷, la différenciation cellulaire ^{20 21} .Pour que cette activation se fasse de manière optimale, il faut que non seulement le ligand soit présent sur l'ECM, mais que ce ligand soit présenté correctement pour interagir avec le récepteur intégrinique ²²⁻²⁴. Il existe d'autre part, au moment de l'activation du récepteur, un couplage physique qui s'établit entre le cytosquelette de la cellule et la partie cytoplasmique du récepteur intégrinique ²⁵. Il se forme alors un véritable pont entre l'ECM, le ligand, le récepteur intégrinique et le cytosquelette. Par le biais de ces connections, des forces sont exercées par la cellule sur l'ECM et le développement de ces forces est indispensable pour l'activation optimale de ces récepteurs^{14,26,27}. Il faut donc que le ligand soit bien fixé pour que de telles forces puissent se développer mais aussi que l'ECM présente une certaine résistance tout en gardant une certaine plasticité pour que ces forces puissent se développer ¹⁴. Dans le cas de cellules contractiles comme les cardiomyocytes, la différenciation est obtenue uniquement avec des substrats de rigidité intermédiaire ²⁸. Les cellules endothéliales se différencient et forment des capillaires ou des structures de type tubulaire sur des substrats souples alors que sur des substrats plus rigides, elles ont tendance à plus s'étaler et à proliférer²⁹.

Afin de réaliser des tissus contractiles, différents types de tissus ont été développés en associant des cellules contractiles à des supports synthétiques ou biologiques (alginate, collagène I, fibrine) ^{4-8,30} Jusqu'à maintenant, aucune contractilité in vitro n'a été rapportée dans les matrices synthétiques ou matrice issues de nanotechnologies utilisant la polymérisation d'oligopeptides (cf. Purametrix®)^{31,32}. Les meilleurs résultats obtenus en terme de contractilité in vitro ont été obtenus avec des matrices de collagène. Il s'agit le plus fréquemment de matrices de collagène obtenues par réticulation physique de fibres de collagène par le procédé DHT (« dehydrothermal treatment »)^{33,34}. Il s'agit cependant d'une technique de réticulation du collagène qui reste considérée comme modérée lorsqu'elle est comparée à d'autres techniques de réticulation par voie chimique. L'avantage des matrices de collagène est leur porosité qui facilite la mise en place des cellules et la diffusion des nutriments et de l'oxygène ³⁵. Cependant la survie des cellules dans ce type de support reste faible, et la différenciation reste partielle avec notamment l'absence de différenciation terminale des cardiomyocytes ⁵ et des cellules endothéliales ³⁶⁻³⁹. Les contractions spontanées dans ce type de matrice restent rares ^{40,41}, et quand elles sont présentes celles-ci sont anarchiques ^{42,43}. Les arythmies sont fréquentes. Afin d'améliorer ces paramètres certains auteurs ont proposés d'utiliser un extrait protidique de membrane basale type Matrigel™ associé à un stimulus physique comme le stress mécanique ⁴⁴ ou l'électrostimulation chronique ^{40,41} et une concentration très importante de sérum xenogénique dans le milieu de culture. Le Matrigel™ est un extrait de protéines extracellulaires de membrane basale de tumeur rénale (cf « Engel Brecht Swarm tumours »). Le Matrigel™ ne peut être employé seul car sa résistance au collapsus est insuffisante et il doit être associé à un support plus rigide synthétique ou biologique. Dans l'état actuel des connaissances, l'utilisation de tissus contractiles obtenus en ayant recours à du Matrigel™ ne peut être appliquée en clinique humaine du fait de l'origine de ce composé. De plus, la présence de Matrigel™ expose à de nombreux effets secondaires. Ce gel limite la libre diffusion des nutriments et le recours à des systèmes de perfusion continue type bioréacteurs devient indispensable ⁴¹. Les troubles de diffusion limitent l'épaisseur du tissu attendu et le rendent impropre au remplacement d'un muscle cardiaque.

Des résultats encourageants ont été obtenus in vivo chez l'animal ^{4,5,7} avec des supports collagéniques cellularisés par des cellules contractiles toujours en présence de Matrigel ⁴⁵. Ces préparations in vivo peuvent continuer à battre pendant plusieurs semaines dans différents sites ^{46,47} et même améliorer la fonction cardiaque systolique après application sur le muscle cardiaque ⁴⁵. On a même pu observer dans certains cas, une synchronisation spontanée entre ces supports et le myocarde de l'animal receveur.

En dépit de ces résultats préliminaires, les matrices de collagène après implantation in vivo induisent une réaction inflammatoire intense conduisant rapidement à la dégradation de la matrice collagénique ⁴⁷. La dégradation accélérée de la matrice du fait de la réaction inflammatoire importante est responsable de la libération locale en grande quantité d'enzymes, de radicaux libres et de différents produits de dégradation qui peuvent également compromettre la survie des cellules associées à ces supports. Les produits de dégradation du collagène eux-mêmes ont été rapportés comme toxiques, notamment pour les cellules contractiles ⁴⁸. De plus, les propriétés mécaniques des supports sont altérées par cette dégradation avant même que les cellules associées n'aient eu le temps d'élaborer leur propre matrice⁴⁹. La survie des cellules dans les supports 3D reste donc généralement faible⁵⁰. Elle est encore plus faible dans les supports collagéniques et la différenciation in vivo des cellules contractiles dans les supports collagéniques reste partielle. Il existe d'autre part une faible angiogénèse dans l'implant qui limite sa fonctionnalité. Pour améliorer cette situation, l'utilisation de Matrigel™ a été indispensable. L'utilisation du Matrigel associé à des matrices synthétiques pour fabriquer un myocarde artificiel a été proposée (cf. Levenberg et al. US patent 20050031598). Cependant Levenberg et al. mentionnent que les propriétés physiques du Matrigel ne sont pas stables in vivo et que les cellules, après s'être initialement différenciées dans ce type de gel se de-différencient rapidement. De plus, la limite de l'utilisation du Matrigel™ pour de telles applications est qu'il s'agit avant tout d'un extrait tumoral à l'origine et donc ne pouvant être utilisé en clinique humaine. D'autre part, le Matrigel™ induit in vivo une réaction inflammatoire très importante qui nécessite l'utilisation de traitements immunosuppresseurs chez l'animal ^{45,47}. La Matrigel™ induit en outre la formation d'une fibrose secondaire cicatricielle peu vascularisée qui compromet à long terme la survie des cellules.

La dégradation des matrices de collagène est généralement réduite par les procédés de réticulation des fibres de collagène. La réticulation par DHT est une méthode classique de réticulation physique ^{33,51}. Ainsi, des hémostatiques employés en clinique sont obtenus par DHT comme la matrice *Ultrafoam*® *(Bard)*). Le collagène initial est soluble dans une solution de 0,5% w/w de collagène dans acide acétique 0.05 M pH 3.5. La préparation est desséchée par le froid de manière contrôlée afin d'obtenir des éponges. La taille des pores et la distribution dépend principalement de la rapidité de la congélation (0.25-1°C/min.), de la température de congélation finale de (-90°C to -5°C) ⁵². Cette éponge est alors soumise à une réticulation par DHT (105°C pendant 16 hr pour une pression inférieure à 100 mTorr) pour introduire des liaisons covalentes entre les chaînes du collagène sans le dénaturer en gélatine ^{53 54}. Bien que ces matrices soient compatibles in vitro avec le développement d'un tissu myocardique (grâce à son architecture, sa résistance, ses propriétés mécaniques, la taille de ses pores compatible avec la diffusion des nutriments) ^{40,55}, cette matrice collagénique obtenue par DHT provoque une réaction inflammatoire intense après son implantation dans les muscles (par exemple muscles squelettiques spinaux) conduisant à sa dégradation en l'espace de quelques semaines.

Il faut donc envisager d'utiliser ou d'associer d'autres méthodes de réticulation du collagène. Afin de limiter cette dégradation le collagène peut être associé à des polymères synthétiques ⁵⁶ ou à des composés organiques ou non organique comme la soie par exemple ⁴⁹.
Tout récemment, une réticulation enzymatique par la transglutaminase a été proposée⁵⁷. La réticulation du collagène peut être obtenue par les cellules et leurs produits naturels. Cette réticulation endogène peut être augmentée par de l'acide ascorbique ou des riboses. Des réticulations peuvent être obtenus par des cellules musculaires produisant une enzyme de type lysyloxidase qui réalise une réticulation entre les groupements amines libres des résidus lysine et les résidus hydroxylysine du collagène. Une autre approche consiste à réticuler les fibres de collagène en faisant appel à des traitements chimiques comme par exemple une réticulation par le glutaraldéhyde ⁵⁸, le diphenylphosphorylazide (DPPA), les carbodiimides ⁵⁹ etc..

La méthode la plus efficace pour réticuler le collagène reste une réticulation chimique par le glutaraldéhyde^{51,58}. Cependant le glutaraldéhyde peut s'auto-polymériser puis se dépolymériser lentement dans la préparation biologique en libérant du glutaraldéhyde libre qui est toxique pour la cellule et donc non compatible avec la thérapie cellulaire.

Ainsi, et bien que la fabrication de tissu cardiaque avec des supports synthétiques ait été proposée (US Patent 20050031598, 20040242469), il n'existe pas actuellement de supports tridimensionnel synthétique ou biologique utilisable chez l'homme et dans lesquels une contractilité aie été démontrée en présence de cellules contractiles ⁶⁰. D'où l'objet de cette invention.

D'autre part, à côté des supports à base de collagène, d'autres supports biologiques à base de fibrine ou de fibrinogène sont en développement et des supports de fibrine ont été proposés pour l'injection de cellules dans le myocarde ⁶¹⁻⁶⁴. Le fibrinogène est soluble et sous l'effet de la thrombine et de calcium, le fibrinogène est transformé en fibrine qui précipite. Les propriétés de ce gel peuvent être contrôlées par la concentration de thrombine et du fibrinogène ⁶⁵. Les propriétés physiques des fibres de fibrines en terme d'extensibilité et d'adaptation aux contraintes sont excellentes ⁶⁶.

La fibrine est un biomatériau qui est utilisé pour la thérapie cellulaire et l'ingénierie de tissu. La fibrine a été proposée pour délivrer des cellules dans des supports synthétiques ou pour fabriquer des tissus cardiaques ⁶⁷, pour injecter des cellules dans le myocarde ⁶⁸ (US patent 200502761631), pour fabriquer des valves cardiaques ⁶⁹, des stents cardiaques ⁷⁰, pour la cicatrisation de tissu bronchique ⁷¹, pour fabriquer des conduits vasculaires ⁷¹.

Il a été montré in vitro que pendant la gélification, différents facteurs de croissance ou des peptides d'adhésion comme le peptide RGD pouvaient être ajoutés au support ⁷². Parmi ces facteurs, La présence de peptide RGD améliore l'angiogénèse dans le support bien que la fibrine contienne déjà le motif RGD ⁷³. Mais il faut noter que dans ces expériences, le RGD n'est pas fixé de manière covalente au support et que d'autre part les gels de fibrine ont tendance à devenir compacts avec le temps avec perte des propriétés mécaniques et le support ne permet plus l'angiogénèse ⁷⁴. L'utilisation de ce type de gel avec des cellules souches pose également certains problèmes car si la concentration de fibrinogène est trop basse le gel se liquéfie et disparaît en quelques semaines. Si la concentration du collagène est trop élevée, les cellules souches ne se différencient plus dans ce type de support⁷⁵. Notons aussi que l'association de fibrinogène avec d'autres composants a aussi été proposée ⁷⁶ et que des hydrogels de collagène et de fibrine ont aussi été développés ⁷⁷. C'est pour les raisons exposées précédemment que nous proposons dans cette invention de modifier le composant collagénique ou le fibrinogène ou la fibrine associés au support avec des molécules d'adhésion fixées de manière covalente.

Parmi les molécules d'adhésion, le motif RGD (Arg-Gly-Asp) est un ligand reconnu par nombre de récepteurs intégriniques présents notamment sur les cellules contractiles ou leurs progéniteurs et sur les cellules endothéliales. Il existe par ailleurs de nombreux autres ligands peptidiques d'adhésion. Ce ligand est normalement présent sur le collagène mais sous une forme non accessible aux cellules si le collagène n'est pas dénaturé (« expression cryptique »). La modification du support collagénique avec des molécules RGD a été proposée dans des applications ²² comme la régénération osseuse et pour la réalisation de tissus dermiques ⁵¹. Dans le domaine thoracique et cardiovasculaire l'association covalente au collagène de motifs d'adhésion comme les peptides RGD n'a jamais été proposée pour l'ingénierie du tissu et la thérapie cellulaire. Lorsque le motif RGD est mentionné, les travaux font état d'un effet délétère du RGD en terme de contractilité, fonction myocardique, rythmicité et sur les forces développées. L'absence de fixation du motif RGD pourrait expliquer l'effet délétère du RGD sur la contraction musculaire rapporté par le groupe de Sarin et al. ⁷⁸ et l'internalisation du motif ⁷⁹ de même que les résultats rapportés par SY. Boateng et al. ⁸⁰. Comme nous le proposons dans cette invention, ces résultats négatifs sont probablement à mettre sur le compte d'une mauvaise utilisation du peptide RGD et dus à l'absence de liaison covalente du peptide utilisé sur le support dans ces différents travaux. Ceci explique l'internalisation du motif dans le travail de S. Balasubramanian et al. ⁷⁹. L'utilisation du motif RGD pour la thérapie cellulaire du coeur a été proposée (US patent 20050271631) mais il s'agit de l'association du RGD à des gels de fibrine et non pas au collagène, le RGD n'est pas fixé de manière covalente et les autres molécules d'adhésions ne sont pas mentionnées. Dans les dispositifs médicaux et chirurgicaux dans le domaine thoracique et cardiovasculaire, il a été proposé d'associer le motif RGD au composant métallique de stent coronaire afin de favoriser l'adhérence de cellules endothéliales circulante et rendre le stent moins thrombogène. Le RGD cyclique a été associé récemment au composant matriciel (polymère de synthèse) d'un stent métallique (en argent) pour diminuer la resténose précoce en favorisant l'adhésion des progéniteurs circulants pour les cellules endothéliales⁸¹. La fixation de peptides d'adhésions sur des supports non plus collagénique mais synthétiques a été envisagée pour l'ingénierie de tissu cardiaque. Il n'en reste pas moins que jusqu'à aujourd'hui aucune contractilité in vitro ou in vivo dans ce type de support synthétique n'a été démontrée ⁶⁰.

Jusqu'à présent, aucune activité contractile n'a été démontrée in vitro et in vivo dans des supports de type synthétiques. Seuls les supports collagéniques ont permis le développement de la contractilité. Une différenciation terminale des cardiomyocytes in vitro n'a été obtenue qu'en présence d'un extrait tumoral de type Matrigel. D'où l'intérêt de supports collagéniques modifiés objet de la présente invention.

Différentes méthodes ont été proposées pour greffer des peptides d'adhésion sur le collagène. Généralement, un groupe réactif du peptide réagit avec un groupement amine présent sur le collagène soit à l'extrémité terminale des chaînes polypeptidiques, soit par les chaînes latérales de la lysine. Par exemple la partie C terminale est transformée en acyl azide. La fixation du peptide au collagène se fait alors par une liaison amide. Cette méthode entraîne la formation d'un grand nombre de produits secondaires, elle n'est pas sélective et ne permet pas d'utiliser de liens espaceurs qui maintiennent à distance convenable de la protéine collagène, la séquence peptidique greffée susceptible de lier le récepteur intégrinique. Une autre approche consiste à modifier le peptide avec un groupement isothiocyanate et de faire réagir ce groupement avec l'amine primaire pour obtenir une liaison de type thioamide. Cette dernière méthode a été proposée par le groupe de Myles et al. ⁸² qui ont proposé de fixer un peptide d'adhésion comme le RGD au collagène avant la fibrinogénèse en utilisant un agent de couplage hétérobifonctionnel de Pierce™ soluble dans l'eau (Sulfo-LC-SPDP) qui permet de lier un peptide contenant un groupement thiol sur un groupe amine primaire du collagène. La dimension linéaire de l'agent de couplage est de l'ordre de 16 angströms.

La technique décrite par JL. Myles et al.⁸² est limitée à l'utilisation de séquences peptidiques d'adhésion liées à un résidu contenant un thiol, ce qui oblige généralement à modifier le peptide en le couplant à une molécule de cystéine. La modification du peptide peut entraîner une interaction différente du peptide avec son récepteur. Les auteurs ont proposés d'effectuer le couplage du peptide avec un collagène en phase liquide avant la fibrinogénèse. Afin de prévenir la fibrinogénèse spontanée, les réactions doivent être effectuée à pH acide. Or le couplage du peptide au collagène est favorisé par un milieu basique ou neutre. D'autre part, la purification des produits de la réaction réalisée en phase homogène est difficile et la séparation des produits secondaires nécessite le recours à plusieurs étapes de purification par chromatographie. Cette difficulté peut être contournée si la réaction est réalisée en phase hétérogène, par exemple sur du collagène insolubilisé par des traitements préliminaires de réticulation. Enfin la technique de Myles JL et al. ⁸² ne permet pas de contrôler l'accomplissement des réactions de couplage et laisse disponibles des groupes aminés capables d'engendrer des réactions de duplication de séquences peptidiques. Il faut ajouter à cela que l'espacement des peptides d'adhésion obtenu par cette méthode n'excède pas 16 angström, ce qui est nettement insuffisant pour présenter les peptides d'adhésions de manière optimale (30-40 angstrôm) ^{23,24}. Nous proposons dans la présente invention une solution à ce problème

La présente invention a pour objet la production d'un environnement biologique naturel tridimensionnel optimisé pour la thérapie cellulaire, ou l'ingénierie de tissu ou l'amélioration de dispositifs pour des applications médicales ou chirurgicales dans les domaines thoracique et/ou cardiovasculaire. Cette invention concerne la fabrication d'un support ou échafaudage (scaffold) tridimensionnel, solide, ces termes pouvant être utilisés l'un pour l'autre. Ce support est constitué d'une éponge de collagène dans laquelle le collagène est réticulé et est par ailleurs modifié en partie ou intégralement par la fixation covalente de molécules d'adhésion associées ou pas à des agents bioactifs qui ne sont pas nécessairement disponibles ou accessibles sur le support ou l'échafaudage d'origine, le collagène étant exempt de tout extrait tumoral. Ces supports tridimensionnels modifiés peuvent être ensemencés et/ou colonisés par des cellules capables de développer leurs potentialités physiologiques.

L'échafaudage tridimensionnel, contient des composants réticulés ou non réticulés. L'échafaudage tridimensionnel peut être formé avant ou secondairement après l'implantation suivant la polymérisation de certains de ses composants. L'échafaudage tridimensionnel peut dans certains cas être injecté. Cette injection peut se faire par exemple dans un vaisseau, dans une lumière bronchique, dans un tissus, dans un espace ou cavité, dans un autre support, dans un dispositif médical utilisé en thérapie cardiovasculaire et thoracique ou combinaison. L'injection peut se faire in vitro et/ou in vivo. L'échafaudage tridimensionnel peut provenir de la polymérisation de quelques composants se produisant spontanément après implantation ou après photoactivation, irradiation comme par l'ultraviolet, irradiation gamma, courant électrique, interaction magnétique, interaction ionique, chimique, température, ultrasons, sel, enroulements hydrophobes/hydrophiles, forces de van der Waals, liaison aromatique-π métal-ligand, pH, concentration, redox, phosphorylation, empilement, forces mécaniques, électromagnétiques ou gravitationnelles. Cette polymérisation ou réticulation peut provenir d'une réaction chimique avec des agents réticulants connus et des dérivés et/ou leurs analogues, dérivés et combinaison, la genipine, aglycone d'acide nordihydroguaiarétique, acide diméthylique, rutérine, acide nordihydroguaiarétique, transformation enzymatique, thrombine, traitement dehydrothermique, réticulation endogène par des cellules et leurs produits biochimiques normaux (tels que la lysine oxydase produite par une cellule ...) ou association de certains de ces procédés.

L'échafaudage tridimensionnel est totalement ou partiellement ou non-biodégradable.

L'échafaudage tridimensionnel est formé dans une phase liquide qui, une fois délivrée, après ou sans activation peut se transformer en phase solide (par exemple solution, pâte, gel, colloïde en suspension, plasma).

L'échafaudage tridimensionnel peut être fait d'un hydrogel constitué d'acides aminés hydrophobes et hydrophiles capables de s'assembler spontanément en structures macroscopiques.

L'échafaudage tridimensionnel peut être un gel ou un agent tensioactif
L'échafaudage tridimensionnel où l'échafaudage est un agent tensioactif ou un « agent intelligent », c'est à dire une matière biologique constituée de structures assemblées spontanément à grande échelle reposant sur des interactions locales au niveau moléculaire.

Dans l'échafaudage tridimensionnel, la construction 3D peut être obtenue par superposition de cultures obtenues sur des échafaudages 2D différents. L'adhérence des cellules à ce support 2D peut être modulable. Ces supports 2D peuvent contenir des collagène/fibrine/fibrinogène modifiés par fixation de molécules d'adhésion.

Plusieurs échafaudages 3D de nature différentes peuvent également être superposés de manière séquentielle ou pas.

L'échafaudage tridimensionnel peut former une matrice de cellules où la construction de tissu artificiel contient des biomatériaux de formes choisies facilitant le regroupement structurel : micro ou nano structures (par exemple les tubes micro ou nano, les nanoparticules, les micro ou nanopores. Les microparticules ou les nanoparticules sont faites du silicium, poly -(acide lactique) mélange d'acide -Co polymère - acide glycolique lactique, cyclodextrine, liposome conjugué ou pas aux nanoparticules quantum dot, magnétite, filaments, des analogues structuraux pour former l'interface extérieure, analogues peptidiques structures β-/ou-α qui forment des filaments ou tubes, d'éponge, de poudre, de conduit, de sphère, de microsphère, de film, micro ou nanofibrilles, membrane de lipide, fibre, maille, matrice, patch, feuille de tissu, ouatine ou combinaison.

Les différents types de support peuvent être associés
L'échafaudage tridimensionnel contient du collagène qui est constitué de collagène (I, II, III, IV, V, VI, VII, XI et d'autres collagènes), ou de l'association de différentes espèces. Le terme « collagène » désigne également collagène insoluble, collagène soluble, atélocollagène préparé en enlevant des télopeptides sur les extrémités des molécule de collagène en utilisant une protéase autre que la collagénase. L'échafaudage tridimensionnel de collagène peut également être un tissu normal d'origine autologue, homologue ou hétérologue. Ce tissu peut être décellularisé ou pas physiquement et/ou enzymatiquement (par exemple avec la collagénase) et/ou chimiquement modifié ou associé. Le collagène peut être purifié à partir de tissu contenant le collagène : autologue, homologue ou hétérologue comme (uretère, péricarde, sous muqueuse comme la sous muqueuse intestinale de porc « SIS » ^{83,84}, vaisseau sanguin, tendon, fascia, derme décellularisé ou pas, aponévrose, membrane type membrane amniotique, dure-mère, valve cardiaque etc....). Ce pourrait être des copies synthétiques du collagène telles que des fibres de polymères ou des peptides formant des fibrilles. Le collagène peut être chimiquement modifié et le produit obtenu par succinylation ou estérification ou formation de carboxyamides, ou désamination des collagènes ci-dessus décrits, mélange de collagène avec des polymères synthétiques tels que poly-acide lactique) (PGA) et/ou poly (DL-lactide-Co-glycolide) (PLGA) et/ou poly (DL-lactide-Co-caprolactone) (PCL), un dérivé de collagène tel que la gélatine, un polypeptide obtenu par l'hydrolyse du collagène, collagène dénaturé par le chauffage. Des polymères synthétiques liés au collagène peuvent être choisis parmi l'acide polylactique (PLA), l'acide polyglycolique (PGA), poly (L-lactique) (PLLA), PLGA, poly (anhydrides) (PA), le polycarbonate (PC) les hydroxy acides, poly ortho- esters (POE), propylfumarates) (PPF), polysaccharides, la polylactone (PL), des poly caprolactones, polyamides, acides de polyamino, polyacétals des polyphosphazènes (PPZ), polycyanoacrylates biodégradables, les polyuréthanes biodégradables (unité centrale), polysaccharides, polypyrrole, polyanilines, polythiophène, polystyrène, polyester (PE), polyuréthanes non-biodégradables, polyurées, poly (éthylène-téréphtalate) (PET), poly (acétate de vinyle d'éthylène), polypropylène, polyméthacrylate, polyéthylène, polycarbonates, poly oxyde d'éthylène, poly alcool de vinyle (PVA), fuseau-tex (polytétrafluoroéthylène), dacron (téréphtalate de polyéthylène), polytétrafluoroéthylène (PTFE), poly-éthylène-glycol (PEG), copolymères décrits ci-dessus, avec l'un des additifs ci-dessus, et mélanges de l'un des polymères, des copolymères, et des additifs entre eux et association de dérivés synthétiques avec les produits biologiques.

Le collagène peut-être seulement un composant de l'échafaudage 3D qui peut contenir des substances synthétiques, inorganiques (comme le verre, Si/Si02, titane/oxyde de titane, or, chrome, cobalt, diamant, platine et hydroxyapaptite, nitinol, acier, silice, streptavidin-biotine, une protéine artificielle comme le latex, le nylon, le catguth, le coton, la toile, le polyester, la soie, le plastique, la céramique, les alliages, le textile, l'avidine, la streptavidine, l'éponge de copolymère - caprolactone-Co-L-lactide renforcée avec du poly-L-lactide, faite en tissu tricoté d'acide hyaluronique (PCLA), l'amidon et n'importe quel mélange), matériaux organiques biologiques (tels que protéoglycanes, glycoprotéines, glycoaminoglycanes, alginate, agarose, l'acide hyaluronique, l'agar,le chitosane, le couple fibrinogène/fibrine, le chitosane carboxyméthylé et le mélange de ceux-ci, gélatine, octasulfate de sucrose, dextrane, cellulose, cellulose méthylée, sépharose, protéine imitée par le Sephadex (comme le latex) ou leur association. L'échafaudage tridimensionnel de collagène peut être le « collagène » contenu dans un dispositif médical utilisé en médecine ou chirurgie cardiovasculaire et thoracique comme par exemple: des valves cardiaques (par exemple des valves fabriquées à partir de cellules autologues y compris cordage, muscle papillaire etc..), des un dispositif médical utilisé en médecine ou chirurgie cardiovasculaire et thoracique comme par exemple: des valves cardiaques (par exemple des valves fabriquées à partir de cellules autologues y compris cordage, muscle papillaire etc..), des bioprothèses cardiaques, des anneaux valvulaires, des tubes valvés, contenus collagéniques de stent coronaires (type « drug eluted stent », « stent cellularisés », « stent biodégradables »), des membranes d'hémocompatibilité, colles, des dispositifs de contention myocardique biodégradables (partiellement totalement ou pas) qui peuvent être cellularisés ou pas et/ou associés (physiquement ou pas) à un autre support qui lui peut être cellularisé, patch, assistance biologique cardiaque visant à favoriser par exemple : la contractilité, la régénération. Un remplacement myocardique, un système de contention pour traiter des conditions myocardiques ou un myocarde pathologique. Un conduit vasculaire spontanément contractile ou sous stimulation électrique par exemple, pacemaker biologique, support pour l'injection cellulaire, matrice ou support de remplacement, support associés à la thérapie cellulaire, prothèse vasculaire, conduit vasculaire (y compris greffon coronaire), endoprothèse, matériels de régénération ou pour la réparation ou remplacement tissulaire, favoriser la cicatrisation, prévenir la déhiscence, favoriser l'étanchéité, prothèse/tissu de remplacement trachéal et ou bronchique, endoprothèse bronchique, conduit trachéal ou bronchique, endoprothèse bronchique, « coil bronchique », « bypass bronchique », tissu pulmonaire, dispositif pour la délivrance d'agent, support 3D collagénique associé à un dispositif médical ou chirurgical etc.....

L'échafaudage peut également comprendre de la fibrine, de haut et de faible poids moléculaire. Les différentes modifications du collagène peuvent s'appliquer à la fibrine. La fibrine et le fibrinogène peuvent être employés l'un pour l'autre. Les différentes formes de « collagène » « fibrinogènes »/ « fibrine » peuvent être associées.

La présente invention a pour objet la production d'un environnement biologique naturel tridimensionnel, optimisé pour la transplantation cellulaire dans le myocarde par exemple grâce à la modification de son collagène par des peptides d'adhésion. Le collagène est une molécule naturelle qui possède déjà certains ligands pour les cellules. Cependant les cellules, en fonction de leur type cellulaire, spécifique avec son environnement et pour un environnement donné de permettre ou pas le développement de tel ou tel type cellulaire.

Les molécules d'adhésion peuvent lier chimiquement le fibrinogène ou la fibrine ou/et être contenues dans le constituant de collagène associé de la préparation dans le cas du gel de collagène et de la fibrine par exemple.

Les molécules d'adhésion incluent poly nucléotides, des peptides d'adhésion qui incluent peptides, polypeptides, protéines ou molécules capables de se lier aux récepteurs cellulaires favorisant l'adhérence cellulaire avec de hautes affinités comme c'est le cas des récepteurs intégriniques et selon les données bibliographiques actuelles. Les peptides d'adhésion peuvent être formés d'acides aminés naturels ou non et/ou analogues. Un ou plusieurs acides aminés du peptide d'adhésion peut être substitué. Les peptides d'adhésion incluent les peptides, polypeptides ou protéines ou molécules (d'origines naturelles ou pas) contenant la séquence d'adhésion initiale. Les termes polypeptide , peptide et protéine peuvent être employés l'un pour l'autre. Ceci comprend aussi l'addition d'une entité chimique telle qu'un groupe d'hydrate de carbone, un groupe phosphate, un groupe farnésyle, un groupe isofarnésyle, un groupe acide gras, un promoteur de liaison pour la conjugaison, fonctionnalisation, ou toute autre modification ou association organique ou inorganique de molécule y compris le polyéthylène glycol (PEG) ou toute autre molécule synthétique. Ces modifications peuvent également inclure la cyclisation du peptide contenant la séquence d'adhésion. Plusieurs peptides d'adhésion peuvent être associés et le peptide peut se rapporter à un peptide individuel ou à une collection de peptides

Les molécules ou les peptides d'adhésion peuvent être modifiés et appartenir aussi à une même molécule ou à une molécule servant d'intermédiaire pour la liaison au support.

Sont en outre inclus dans le peptide, les peptides d'adhésion qui visent à interagir avec le récepteur d'adhésion lui-même, avec les co-récepteurs (impliqués non nécessairement dans l'adhésion). La liaison à ces co-récepteurs modifie et/ou l'adhésion et/ou l'activation et/ou la transduction du récepteur.

Selon la présente invention, le peptide d'adhésion pourrait être avantageusement le peptide RGD correspondant à l'arginine (R) - la glycine (G) - l'acide aspartique (D), motif de reconnaissance de l'intégrine sur la fibronectine décrite par Pierschbacher et al. ((« RGD linéaire »)(*Pierschbacher MD. et al. 1984*)⁸⁵), ou sur la vitronectine comme décrite par Plaff M. et al. ((« RGD cyclique ») *(Plaff M. et al. 1994*)⁸⁶). Le « motif RGD » inclut également tous les ligands peptidiques qui interagiront avec l'un des récepteurs intégriniques αᵥβ1, αᵥβ3, αᵥβ5, αᵥβ6, αᵥβ8, α_{llb}β3, α₄β1, α₄β₇, α₅β₁, α₈β₁. D'autres peptides d'adhésion tels que les peptides PHSRN ou domaine obligatoire protéoglycane de la matrice extracellulaire (domaines obligatoires d'héparine) basés sur X-B-B-X-B-X ou X-B-B-B-X-X-B-X, modèles d'ordre de B-B-X-B, où B est un aminoacide de base et X est un hydroxy aminoacide, YIGSR (*Iwamoto et autres. 1987*)⁸⁷ et IKVAV (- Ile-Lys-Val-Ala-Val-) *(Tashiro et al. 1989*)⁸⁸ RYWLPR ou RNIAEIIKDI *(Liesi P. et al. 1989)⁸⁹* de la laminine, REDV (*Massia SP et al. 1992*)⁹⁰, PHSRN (- Pro-His-Ser-Arg-Asn) (*Aota* S. *et al. 1994*) ou KNEED (*Altroff H. et al 2001, Wong JY et al. 2002*)^{91,92} ou EILDV de la fibronectine, domaine obligatoire protéoglycane des protéines d'adhésion telles que KRSR (Dee *KC et al. 1998* ; *Rezania A. et al. 1999*)^{93,94} ou FHRRIKA (*Rezania A. et al. 1999*)⁹⁵, séquence de VAPG et de KQAGDV de l'élastine (*Mann et West et al. 2002*)⁹⁶, GFOGER du type I de collagène (*Emsley J. et al. 2000)⁹⁷,* DEGA.

Dans un cas particulier de l'invention, le ligand contient le motif RGD présent sur les protéines de l'ECM comme la fibronectine ou vitronectine mais de très nombreux autres ligands peuvent être associés de la même façon (par exemple des séquences peptidiques dérivées de la laminine etc...) y compris ceux déjà présents sur le collagène.

La présente invention fournit également une méthode simple, partant d'un échafaudage constitué totalement ou en partie de collagène et contenant des groupes fonctionnels disponibles tels que thiol ou amine ou carboxyliques, pour lier des molécules d'adhésion ou des molécules d'intérêt ou bien pour les libérer in situ de façon contrôlée. Cette méthode tend à améliorer la présentation de ces molécules ou des agents biologiques (tels que protéoglycanes, des facteurs de croissance ou des cytokines) vis-à-vis des récepteurs membranaires de cellules capables d'habiter la matrice collagénique modifiée.

Dans une application particulière de l'invention, l'agent de couplage hétérobifonctionnel est sulfo-LC-SPDP.

L'invention est caractérisée par une présentation spatiale améliorée du peptide ou du facteur de croissance, une diminution de la réaction secondaire ou du couplage inter et/ou intramoléculaire indésirable, qui améliorent l'efficacité globale du couplage tandis que le contrôle à chaque étape du procédé est possible.

La technique employée utilise un même réactif hétérobifonctionnel fixé d'une part sur le peptide de d'adhésion et, d'autre part, sur le support collagénique. La fonctionnalisation du support collagénique permet, en outre, de présenter le peptide d'adhésion à son substrat à une distance de 36 angström, distance qui est connue pour être la distance optimale pour la présentation des peptides d'adhésion (entre 30-40 angström)^{23,24}. La réaction permet de coupler de manière unidirectionnelle le peptide sur le site actif du support sans duplication de peptide. Toutes les étapes intermédiaires de la réaction peuvent être contrôlées par simple analyse spectrophotométrie du milieu. Si le couplage de la molécule est effectué sur un support solide, aucune chromatographie n'est nécessaire pour éliminer les produits intermédiaires qui peuvent être éliminés par simple lavage. De plus, la réaction peut être conduite à pH neutre ou alcalin, conditions les plus favorables pour la conjugaison. La molécule peut contenir des groupes masquant permettant des réactions successives au substrat et aux ligands. Les méthodes de liaison se rapportent à des molécules réagissant avec chaînes latérales des acides aminés (par exemple amine ou thiol ou alcool ou aldéhyde ou amide ou groupes acides ou aux combinaisons de ceux-ci) dans les substrats (par exemple l'échafaudage 3D et les peptides-RGD choisis). La méthode s'applique si la liaison a lieu avant ou après la fibrinogénèse du collagène. Cette méthode s'applique aussi à toute méthode pour coupler les groupements aminés du collagène en utilisant un agent de couplage hétérobifonctionnel contenant aussi un groupement thiol masqué permettant d'activer séparément le peptide et le collagène. Ces liaisons chimiques peuvent aussi être formées de façon irréversible par d'autres moyens tels que radiations ionisantes, initiation par des radicaux libres, ou d'autres réarrangements moléculaires. Un résidu maléimide peut être utilise à la place d'un groupe pyridyldisulfure. L'invention propose aussi une méthode pour lier le motif RGD en tant que dérivé soufré à un échafaudage métallisé dans la mesure où le métal serait recouvert d'une mince couche métallique présentant une forte affinité pour les atomes de soufre du peptide RGD fonctionnalisé pour le couplage. D'autre part la liaison covalente de la molécule d'adhésion sur le support ou sur un agent fixé sur ce support peut être ultérieurement clivée par des réactions chimiques, enzymatiques, thermiques, mécaniques ou association de celles-ci.

La présente invention décrit des procédés pour augmenter la biocompatibilité des échafaudages de collagène en utilisant des réactifs chimiques non toxiques permettant la réticulation du support, la fixation des molécules d'intérêt et la croissance in situ des cellules favorisée par la présence de molécules d'adhésions comme des peptides d'adhésion ou facteurs précédemment mentionnés.

Jusqu'ici, la réticulation par voie chimique la plus efficace pour le collagène a été obtenue avec le glutaraldéhyde. Le glutaraldéhyde peut se polymériser et s'hydrolyser par la suite, libérant le glutaraldéhyde libre qui est cytotoxique. Un tel échafaudage ne peut donc pas être employé pour l'ingénierie de tissu.

Un mode de fixation consiste donc à obtenir une fixation irréversible du glutaraldéhyde par neutralisation des groupes aldéhyde résiduels par une réticulation irréversible d'un glycosaminoglycane comme héparine ou héparine sulfate. Dans la première approche, le tissu est fixé par du glutaraldéhyde et les groupements aldéhyde libres sont neutralisés de manière irréversible en utilisant un glycosaminoglycane (GAG) comme l'héparine/héparine sulfate, chondroïtine sulfate, dermatane sulfate. Les GAG sont connus pour réguler le degré d'hydratation des matrices mais servent aussi de ligand pour beaucoup de facteurs de croissance. Lee et al. ont rapportés une méthode pour limiter les groupements aldéhydes réactifs après fixation avec le glutaraldéhyde en bloquant avec de l'amino polyethylene oxy sulfonate, (cf. NH.sub.2-PEO-SO.sub.3 (cf. *Lee et al. (2001*))⁹⁸ ou avec de l'héparine. Dove JS (US patent 20060217805) ont proposé de fixer le tissu avec du glutaraldéhyde puis de traiter le tissu avec un agent réducteur capable de réduire le glutaraldéhyde, probablement les imines et les groupes carboxylique sur les tissus fixés. Cependant la méthode employée pour fixer un tissu est difficilement transposable à un support collagénique notamment plus fragile. Il semble plus approprié d'utiliser des agents réducteurs moins drastiques pour réduire les groupes imines formés lors de la réaction des groupements amine avec le glutaraldéhyde.

Dans le but d'améliorer la compatibilité, une autre approche viserait à utiliser des agents réticulants différents du glutaraldéhyde. On sait de *Y. Chang et al. (2002); HC Liang (2004); CC Tsai et al. (2001)*^{99,100}*) (*US patent 20050013802*)* que la Genipine a été proposée pour réticuler des tissus natifs ou décellularisés. L'utilisation de genipine n'a pas contre pas été proposée comme agent pour fixer des support collagénique reconstitués à partir de fibres de collagène et destinés à être cellularisés. Les seules publications relatant l'utilisation de la Genipine dans le domaine cardiovasculaire concernent le contrôle de la dégradation du composant collagénique de dispositifs médicaux tels que « drug eluted stent » (US Patent 20050123582*).* Il ne s'agit pas dans ce cas de stent cellularisé, le composant « drug » ayant pour objectif de contrôler la prolifération cellulaire par sa toxicité. Dans la présente invention nous proposons d'employer la Genipine en association ou non avec d'autres traitements physiques tel que UV, irradiation par faisceaux ionisants, déshydratation et traitement par la chaleur (*dehydrothermal crosslinking (DHT) KS Weadock et al (1996*)³³ pour stabiliser la matrice artificielle de collagène (collagène, gélatine ou chitosane) pour l'ingénierie de tissu après ou avant association avec des peptides d'adhésion tels que RGD.

La présente invention se distingue du brevet de Mooney et al 6,642,363 US Patent (2003) dans la mesure où elle concerne la fabrication d'un tissu artificiel pour des applications cardiovasculaires comportant essentiellement des myocytes semés dans une matrice de collagène biodégradable tandis que les auteurs cités décrivent la fabrication d'un tissu à partir de chaînes de polyalginates.

La présente invention se distingue du brevet de Barerra et al US Patent 5,399,665 (1995) qui utilise des polymères fonctionnalisés mais pas de collagène.

La présente invention se distingue du brevet de Hai-Quan, Mao et al. US Patent 20050058692 qui utilisent le collagène comme biomolécule ligand d'une matrice de polymère synthétique

Dans la présente invention nous fournissons pour la première fois la preuve que la différenciation terminale de la cellule contractile peut être obtenue dans des matrices collagéniques par la simple fixation sur ces matrices de peptides d'adhésion tels que le peptide RGD. L'activité contractile spontanée ou obtenue par stimulation est augmentée ainsi que le seuil électrique, ces facteurs sont très importants car, après implantation, il faut que le myocarde implanté soit stimulé par le muscle cardiaque natif afin que les contractions se fassent de manière cohérente. Nous proposons également une manière de retarder la réponse inflammatoire et la réponse immunologique contre le greffon par des approches de réticulation chimique non toxique. Le peptide RGD fixé à la matrice collagénique favorise également l'angiogénèse in vitro et in vivo. On sait, en effet de Levenberg S. et al (2005) ¹⁰¹ que la prévascularisation ou l'angiogénèse locales sont des éléments importants pour promouvoir la fonctionnalité de l'implant et la survie des cellules associées

L'agent peut être un agent chimique, physique, biologique ou combinaison. Il peut être par exemple un facteur de croissance, une force mécanique (y compris et pas exclusivement les interactions extérieures, l'effort de cisaillement et l'effort mécanique), des stimuli électriques, une tension électrique, champ magnétique, une biomolécule et une petite molécule ou sa combinaison.

Sont d'un intérêt particulier les agents qui contrôlent l'adhésion des cellules, la survie, la prolifération, l'apoptose, la différenciation. Il peut s'agir de d'agents qui contrôlent l'angiogénèse, l'ischémie, la dégradation, le renouvellement, l'immunogénicité de la matrice extracellulaire, la réaction inflammatoire ou immunitaire, l'installation des cellules dans leur environnement (cellular homing), la prolifération tumorale, la fonction myocardique, vasculaire ou trachéobronchique et pulmonaire. Pour les agents biologiques l'agent ou son ou ses récepteurs peuvent être employés indifféremment.

La dénomination « agent » comprend aussi : collagène, fibrine ou fibrinogène, cytokine, chemokine, eicosanoides, glycoprotéines, glycosaminoglycane (par exemple héparine/sulfate d'héparane (par exemple inclus ci-dessus syndecane 1; 2 ; 3 ; 4), chondroïtine (inclus ci-dessus pas exclusivement chondroïtine 4 et 6)/sulfate dermatane ou sulfate keratane), agents keratane et chimiotactiques , un récepteur de facteur de croissance, une enzyme, une hormone ou son récepteur, un facteur angiogénique, un facteur promoteur de vascularisation ou facteur l'empêchant, un antigène vaccinique, un anticorps, un facteur de coagulation, une protéine de normalisation, un facteur de transcription, facteur de différenciation, un récepteur, ADN, cDNA, ADN-aptamers, toxine, une protéine structurale, une molécule d'adhésion, drogue, médicament, chimiothérapie, antibiotiques, agents antifungiques et antibactériens, agents antimicrobiens antiviraux, antinfectieux et tout fragment, variante ou combinaison.

Dans un mode de réalisation spécifique le dépôt de cet agent fournit des localisations pour y déposer un matériel biologiquement actif pour le traitement de l'ischémie ou le dysfonctionnement cardiaque tel que agent bloquant alpha ou bêta adrénergique, ou agonistes, , activateurs d'AMP kinases, inhibiteurs d'angiotensine converting enzyme (ACE), angiotensine II receptors, agents antiarrythmiques, agents anticoagulants, agents anti-aggrégation plaquettaire, agents antidiabétiques, antioxydants, agents anti-inflammatoires, sequestrants d'acide biliaire, bloquants de canaux calciques, antagonistes du calcium, CETP inhibiteurs, régulateurs de cholestérol et de lipides, drogués qui bloquent la conversion de l'acide arachidonique, diurétiques, agents de remplacement des oestrogènes, agents inotropiques, analogues d'acides gras, inhibiteurs de la synthèse des acides gras, fibrate, histidine, dérivés de l'acide nicotinique, nitrates, activateurs de la prolifération des peroxisomes agonistes or antagonistes des récepteurs, ranolzine, statines, thalidomide, thiazolidinediones, agents thrombolytiques, vasodilatateurs et vasopresseurs et autres listes de facteurs SDF-1, facteur mesenchymal homing de cellules tel que l'alpha MCP-3, OEB, ligand de TGF alpha ou bêta de la superfamille, les chemokines de LIF, de BMP, les récepteurs de BMP ou etc. la molécule de signalisation telle que le smad ou l'Idb3, le BMP2, le BMP4,...., inclus également mais pas exclusivement, la drogue, le facteur de croissance est choisi parmi l'activine-A (ACTE), acide retinoïque (RA), facteur épidermique de croissance, la protéine morphogénétique de l'os, Facteur de croissance tumoral TGF comme « TGF-.beta », HGF (facteur de croissance de l'hépatocyte), PGDF (facteur plaquette-dérivé de croissance, albumine, oxygénase de heme, LDL (protéine de faible densité), alpha. (facteur de nécrose de tumeur), le facteur de croissance d'insuline (IGF-I et/ou II), facteurs de croissance de fibroblastes, facteurs de croissance de nerf (NGF), facteur morphogène de muscle (MMF), facteur dérivé par stroma d'alpha-chemokine (SDF) - 1, VEGF (facteur endothélial vasculaire de croissance de cellules), facteur de croissance de fibroblaste (FGF) ou récepteurs, facteurs hématopoïetique de croissance, héparine, sulfate de héparane, sulfate de chondroïtine, glycosaminoglycane, anticoagulant, agents thrombolytiques, agents antifibrinolytiques, agents antiplaquettaires, facteurs de la coagulation, l'activateur plasminogène de tissu (t-PA), thrombomoduline, le kinogène de haut poids moléculaire, AT-III, inhibiteur de C&-estérase, le facteur H, l'érythropoïétine, SCF (facteur de croissance de cellules de souche), G-CSF (facteur de croissance de granulocyte), GM-CSF (facteur stimulant de Granulocyte-macrophage), facteur du complément, le facteur de croissance dérivé par plaquette (PDGF), Monocyte chemoattracted protein-1 (MCP-1), facteur épidermique de croissance (EGF), hormone parathyroïde (PTH), transporteur de sérotonine ou de récepteur de sérotonine ou de sérotonine ou antagoniste d'agoniste de récepteur de sérotonine, endoglobulines, facteur endothélial de croissance de cellules, facteur stimulant, CXC d'angiogénèse de cellules endothéliales chemokines, HIF-1alpha, Angiogénine, facteur obligatoire de croissance d'héparine, facteurs de croissance de peptide, insuline, IGF (facteur de croissance d'insuline), oestrogène, hormone humaine de croissance (hGH), Follistatine, Proliferine, prostaglandine, interleukines ou récepteur, globines, immunoglobulines, antigène histocompatibilité type Antigène HLA, anticorps catalytiques, facteurs de nécrose de tumeur, chemokines, agent immunosuppressif, anti-inflammatoires, antinéoplasiques, antibiotiques, agents antifungiques et antibactériens, agents antimicrobiens anti viraux, antinfectieux, leptines, interférons, facteurs stimulants de colonie, V-MAP, Angiopoietin, récepteur de déclenchement du peptide de latence (tel que LAPβ1, LAPβ3) pour les facteurs de croissance ou les cytokines, VEGF-1, VEGF-2 (KDR), CTGF, Tie-1, Tie-2, SCA, CD133, CD34, CD43, Ephedrines, protéines qui contrôle la dégradation de la matrice comme les proteines type métalloprotidases(MMP), MMP 2, Del 1, alpha HIF-1, protéine chemotractant de Monocyte (MCP-1), agents régénérateurs oligomères (RGTA), nicotine, glycosamino glycane comme l'héparine/sulfate de heparane ou le sulfate de chondroïetine/dermatane/keratane, laminine ou peptides contenant le motif ligand YIGSR de laminine, la séquence d'IKVAV, de VAPG de l'élastine, les domaines de liaison de l'héparine basés sur X-B-B-X-B-X ou X-B-B-B-X-X-B-X, séquences B-B-X-B, où B est un aminoacide et X est un hydroxyaminoacide, protéoglycanes ou les molécule qui visent à lier le protéoglycanes (tel que le domaine héparine liant de Hepll de la fibronectine par exemple), REDV, PHSRN, RGD, FHRRIKA, adénosine et inosine seule ou en association, molécule d'adhésion ou récepteur pour la molécule d'adhésion (y compris pas exclusivement des ligands identifiés par des récepteurs d'intégrine mais également d'autres récepteurs ou co-récepteurs qui médient l'adhésion cellulaire).

L'agent associé peut être lié chimiquement, adsorbé, absorbé ou libre.

L'agent peut-être contenu dans le volume ou à l'extérieur de l'échafaudage 3D dans un tissu ou un échafaudage externe ou dispositif médicochirurgical en tant que défini pour l'échafaudage secondaire et dont le but est de traiter le premier échafaudage ou le tissu à son contact.

L'agent peut être présent dans l'échafaudage 3D in vitro ou in vivo avant, pendant ou après la période d'implantation de l'échafaudage 3D.

L'agent a pu être délivré par un système externe de contention, dispositif endovasculaire ou endobronchique, dispositif tissulaire ou endocavitaire.

L'échafaudage crée dans le tissu des niches localisées pour y déposer un matériel biologiquement actif. Le tissu sert à concentrer la liaison du matériel biologiquement actif tel que les drogues qui sont introduites localement ou systémiquement. L'échafaudage peut également servir à la libération d'agent bioactif associé à l'échafaudage.

Un contingent cellulaire peut être associé ou non au support tridimensionnel de collagène modifié. Dans certains cas, le contingent cellulaire peut provenir de la colonisation du support par les cellules de l'hôte. Les cellules peuvent être des cellules embryonnaires, foetales, néonatales ou adultes. Les cellules peuvent être des cellules souches, des cellules souches prédifférenciées, progéniteurs ou des cellules différenciées ou une combinaison de celles-ci. Les cellules peuvent avoir une origine autologues, cellules homologues, hétérologues comme des cellules de mammifères.

Les cellules peuvent être manipulées génétiquement. Le composant cellulaire où des cellules ont été manipulées pour produire un agent bioactif. Le composant cellulaire où les cellules ont été manipulées ex vivo ou in situ ou en combinaison

Le composant cellulaire peut être obtenu par la fusion de différents types de cellules de mammifères ou non mammifère. Les cellules peuvent avoir des degrés différents de différenciation, être activées ou pas, être d'âge différents, manipulées ou pas ou en combinaison.

Les différents types de cellules peuvent être associés.

Un ou plusieurs types de cellules peuvent être présents dans l'échafaudage 3D Les différents types de cellules ne sont pas forcement associés et présent au même moment dans l'échafaudage. Les cellules peuvent être associées initialement à l'échafaudage in vitro ou in vivo. Les cellules peuvent être cultivée dans l'échafaudage in vitro avant implantation. Les cellules peuvent être associées secondairement à l'échafaudage. Afin de coloniser le support, Les cellules peuvent par exemple être injectées directement dans l'échafaudage, à proximité, ou à distance. Le support peut ainsi être par exemple injecté par voie endovasculaire et les cellules injectées de manière concomitante ou secondairement. La présence des cellules in vivo peut dans certains cas également précéder la mise en place de l'échafaudage. Dans certains cas, le contingent cellulaire associé peut provenir de la colonisation du support par les cellules de l'hôte. L'association des différentes combinaisons d'utilisation est possible.

La méthode inclut mais pas exclusivement des cellules à potentiel contractile, rythmique ou angiogénique comme les cellules souches embryonnaires humaines, les cellules souches embryonnaires dérivées dés blastocytes humains de SCNT (transfert nucléaire), la cellule souche d'adulte purifiée de tissu différencié : du foie, du pancréas, du coeur, poumon, de la moelle osseuse, tissu musculaire (lisses, cardiaques, squelettiques etc..., les progéniteurs pour les cellules endothéliales ou contractiles isolées de tissu foetal, néonatal ou d'adulte tel que la cellule de Sca1+/- isolée du pancréas, du coeur, du foie ou de tout autre tissu foetal, cellule pre-différenciée in vitro ou in vivo, le myoblaste squelettique ou le myocyte, le cardiomyocyte (foetal, néonatal, adulte), progéniteurs pour le cardiomyocyte ou le myocyte, cellule mesenchymale de moelle humaine, de stroma, cellules hematopoïetiques, cellules progénitrices isolées à partir de sang de cordon ombilical, leucocytes du sang et cellules progéniteur de sang pour les cardiomyocytes ou les cellules endothéliales (telles que les cellules CD34+, AC133+), cellules amniotiques, cellules de placenta, cellules de trophoblaste, cellules basales, les cellules de tissu adipeux ou les cellules dérivées, cellules purifiées à partir de l'epiploon comme les cellules mésothéliales épiploïques, cellules periostéales, cellules perichondriales, fibroblastes, cellules neuronales, cellules hippocampales, cellules épidermiques, cellules dermiques, keratinocytes, cellules granulaires, cellules de système immunitaire, des cellules pré-différenciées (en employant des traitements électriques, l'hypoxie, l'hypothermie ou la congélation, par la stimulation ou le retrait de certaines chemokines, des produits chimiques, biologiques et d'autres traitements physico-chimiques).

Dans les exemples suivants seront décrites les différentes phases d'un mode particulier de réalisation d'une matrice de collagène modifié de même que les caractéristiques des préparations ainsi obtenues.

### EXEMPLE 1

Couplage covalent de molécules d'adhésion telles que les peptides RGD/RGE au support collagénique ou agents biologiques tels que protéoglycanes, des facteurs de croissance ou des cytokines si l'échafaudage est fait de collagène

### EXEMPLE 1

Couplage covalent de molécules d'adhésion telles que les peptides RGD/RGE au support collagénique ou agents biologiques tels que protéoglycanes, des facteurs de croissance ou des cytokines si l'échafaudage est fait de collagène et contient des groupements accessibles thiols, amines ou des groupes carboxyles

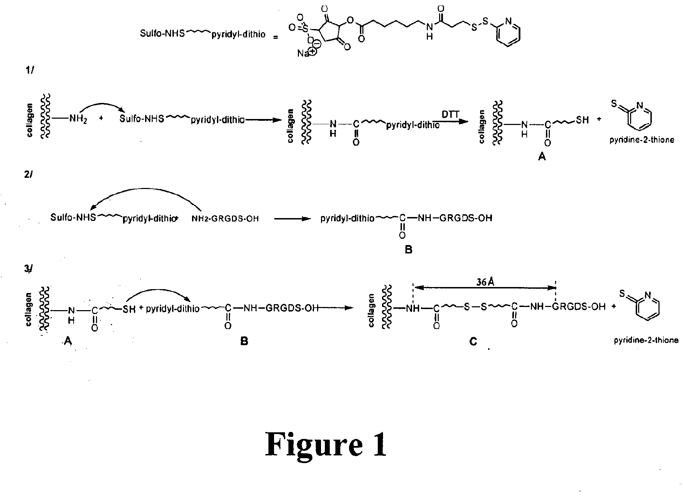

Le principe de la réaction chimique de couplage est illustré dans la figure 1. Le couplage décrit ici permet de présenter le motif RGD à une distance moyenne du support ici une matrice de collagène de 30-40 Angstrôms, élongation favorable à l'interaction maximale de la séquence peptidique avec le site récepteur de l'intégrine (cf. Beer JH. et al. 1992, Craig WS. et al. 1995)^{23,24}.

Sont utilisés des supports collagéniques et avantageusement des supports poreux réticulés par DHT comme Ultrafoam@, matériaux déjà autorisés en clinique comme hémostatiques : les feuilles d'Ultrafoam@ de 5 mm d'épaisseur (2.5 mm après réhydratation en PBS) (Davol Inc., Cranston, RI) proviennent de fibres de collagène de type I et type III de boeuf réticulé par DHT. Les matrices, après réhydratation sont découpées en disques de 8 mm de diamètre et 2.5 mm d'épaisseur en utilisant des punchs pour biopsie cutanée. La concentration du collagène du support après réhydratation est de l'ordre de 20 mg/cm³. La taille des pores du support s'échelonne entre 30 et 200 µm (H. Park 2005)⁵⁶. Les peptides linéaires RGD (par exemple GRGDS) or RGES (Sigma Aldrich) ont été fixés de manière covalente à la matrice collagénique après formation de la trame collagénique en utilisant un agent réticulant hétérobifonctionnel soluble dans l'eau, le (6-[3'-2-(pyridyldithio)-propionamido] hexanoate (cf. (Sulfo-LC-SPDP) (Pierce Biochemical (Rockford, IL, USA)).

Cet agent réticulant hétérobifonctionnel (nommé aussi crosslinker) réagit par son groupement *N*-hydroxysuccinimide avec les groupements amine présents sur le collagène L'autre extrémité du crosslinker qui contient le groupement 2-pyridyl disulfure réagit, après conversion en un groupement thiol par le dithiothreitol, avec le peptide d'adhésion activé. Ce peptide, avantageusement GRGDS, a été couplé extemporanément avec le crosslinker Sulfo-LC-SPDP sur la partie N-terminale du résidu glycine ou sur le résidu arginine dans le cas de RGES. Par exemple, GRGDS ou RGES (solutions aqueuses stériles) (0,1-10 mg/ml) sont mis à réagir avec Sulfo-collagénique réhydratée en PBS pH 7 . L'excès de crosslinker est enlevé par lavages successifs de la matrice dans le PBS. Les matrices ainsi modifiées sont traitées par Dithiothreitol (DTT) (12 mg / ml dans PBS) et DTT est éliminé par lavages. Dans la réaction finale, les matrices collagéniques traitées précédemment (10 mg) réagissent avec Sulfo-LC-GRGDS (0; 0.5; 1 and 2 mg) ou Sulfo-LC-RGES (1 mg), 48 heures dans PBS. Le degré de substitution est contrôlé en changeant les quantités relatives des différents réactifs par rapport au collagène et surtout par la mesure spectrophotométrique de la pyridine-2-thione issue du clivage du crosslinker (absorption molaire spécifique = 8.08 x 10³ M⁻¹ cm⁻¹ à 343 nm) (**figure** 1). Les préparations sont maintenues stériles et gardées à 4°C.

### EXEMPLE 2

Utilisation de supports collagéniques fonctionnalisés avec des molécules d'adhésion pour fabriquer un tissu contractile

### Cellules contractiles

Différents types de cellules ont une activité contractile avant ou après une différenciation complète comme par exemple les cellules musculaires lisses, les cellules musculaires squelettiques ou des cardiomyocytes. Des cellules souches embryonnaires prédifférenciées ou pas (agent de différenciation pouvant être par exemple un ou une combinaison de facteurs de croissance. Le retrait ou l'inhibition de certains facteurs du milieu de culture comme par exemple des facteurs de croissance type FGF, TGF beta, BMP-2, SDF1, facteurs physiques comme l'hypoxie, l'electrostimulation, la congélation, le stress mécanique etc...) ont également montré leur capacité à favoriser la différenciation vers des cellules contractiles. Des cellules de la moelle (cellules hematopoiëtiques ou cellules mésenchymales), des cellules isolées du sang circulant (incluses également les cellules isolées à partir du sang du cordon ombilical). Des cellules à potentiel contractile ont également été isolées à partir de tissus différenciés (foetaux ou adultes), tissus musculaires (comme des myoblastes ou des cardiomyoblastes), des progéniteurs isolés du tissus adipeux ou épiploïques, de tissus différenciés foetaux ou adultes (comme par exemple le foie, pancréas, myocarde, poumon etc.), liquide amniotique, cellules modifiées génétiquement etc.

### Mise en place des cellules dans le support collagénigue :

Dans cet exemple des cellules contractiles différenciées classiquement utilisées, des cardiomyocytes néonataux de rat (obtenus par digestion de ventricule de coeur de rat nouveau né dans les deux jours qui suivent la naissance) ont été mises en place dans des matrices collagéniques fonctionnalisées ou pas avec des peptides d'adhésion. Les matrices collagéniques ont été obtenues par DHT. Dans l'exemple suivant il s'agit d'une matrice commerciale type Ultrafoam™ (Bard) mais d'autres types de matrice ayant un composant collagénique peuvent être utilisées. Un nombre important de cellules 1 10⁷ cellules/cm3 de matrice collagénique ont été ainsi mises en place dans la matrice. A titre de comparaison, dans le myocarde, le nombre de cardiomyocyte rapporté au volume de tissu myocardique est de 0.5-1.10⁸ cardiomyocytes /cm³ (*ref. M. Radisic et al. 2003*)¹⁰²*.* Les supports collagéniques modifiés avec les peptides d'adhésion (dimension : 8 mm de diamètre x 5 mm d'épaisseur) ont été ensemencés avec 2 x 10⁶ cardiomyocytes afin d'obtenir une concentration de 1.5 x 10⁷ cellules/cm³. Juste après le dépôt des cellules, les matrices ont été centrifugées à 1000 tours/min. pendant 6 min. afin d'améliorer l'efficacité et l'homogénéité de la distribution cellulaire. Les cellules non attachées présentes dans le culot ont été redéposées sur la face supérieure de la matrice.

### Culture des échafaudages cellularisés

Les matrices cellularisées ont été transférées ensuite dans des plaques 12-puits et cultivées en condition statique sans avoir recours à des bioréacteurs et en utilisant un milieu de culture classique 2ml de DMEM (Dulbecco's modified Eagle's medium) enrichi avec 10% de sérum de veau foetal sans utiliser de sérum de cheval comme cela est habituellement nécessaire. Après 24h. le milieu de culture a été remplacé pour du DMEM ne contenant que 5% de sérum de veau foetal et contenant de la transferrine (10 mg/ml), de l'insuline (1 mg/ml) et du sélénium. Le milieu de culture a été changé 2 fois par jour jusqu'au 8^{ème} jour. Il faut insister sur le fait qu'aucune matrice extracellulaire type Matrigel™, des stimuli physiques comme une électrostimulation chronique or un stress mécanique, l'utilisation de bioréacteur ou de fortes concentrations de sérum xénogénique de type de sérum de cheval n'ont été utiles pour obtenir les résultats obtenus.

### Histologie

Les matrices cellularisées ont été fixés au 8^{ème} jour dans le formol à 10% puis incluses en paraffine pour une histologie classique Des sections transverses de 5 µm ont été marquées avec une coloration par HES (haematoxyline-eosine-safran) pour l'analyse architecturale et pour étudier le nombre et la distribution des cellules. Une certaine partie des matrices cellularisées a été également congelée dans l'azote liquide pour effectuer des immunomarquages. Après décongélation, les sections ont été perméabilisées avec du triton X-100, saturées avec du PBS contenant 3% d'albumine sérique de boeuf. Les anticorps primaire puis secondaire conjugués à un marqueur fluorescent (Alexa) ont été alors incubés avec les sections. Les matrices RGD modifiées ont été ainsi incubées avec un anticorps de souris dirigé contre l'actinine alpha (concentration d'utilisation dilution 1 :500 (Sigma)) et un anticorps secondaire antisouris conjugué à alex-546 (Molecular Probe) (dilution 1 :300). Les noyaux des cellules ont reçu un marquage spécifique en DAPI.

La mise en place de cardiomyocytes néonataux de rat dans une matrice collagénique modifiée avec des peptides d'adhésion permet de réaliser un implant qui se contracte spontanément après ≈2-3 jours de culture in vitro. Au 8^{ème} jour, une activité contractile a été observée dans 50% des implants collagéniques en l'absence du motif RGD et dans 80% en présence (p=ns). De manière intéressante, les battements spontanés sont plus rapides en présence du motif RGD 141±17 versus 61±26 (différence significative p<0.05. Quand ces battements sont présents, ils sont également plus réguliers dans les matrices collagéniques modifiées.

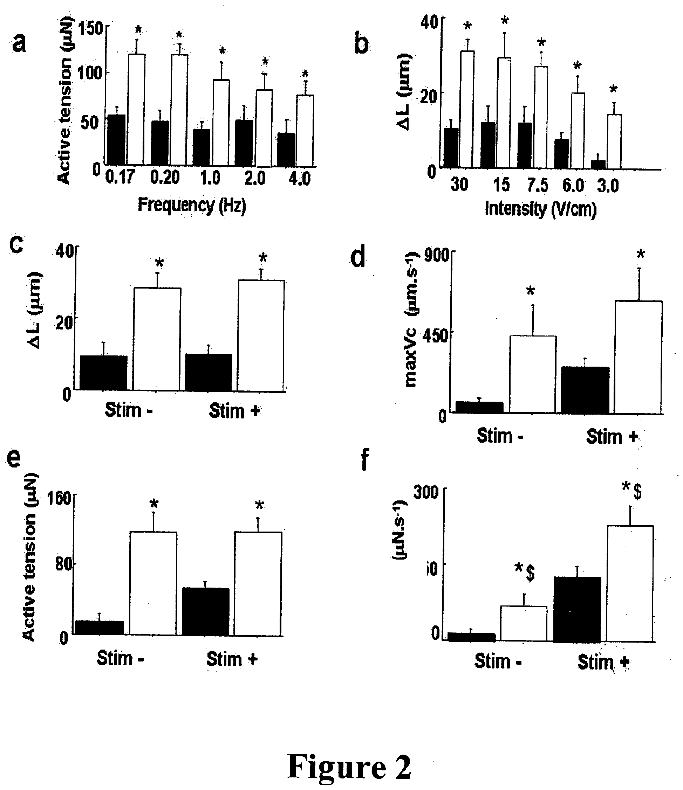

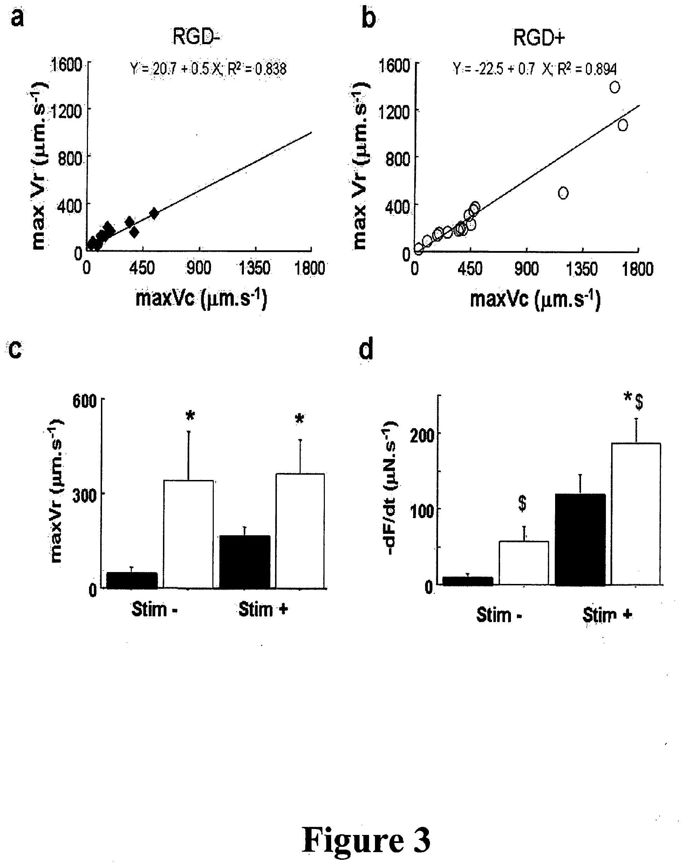

Les différents paramètres de contractilité sont présentés dans la **figure 2** pour ce qui est des paramètres de contraction ou dans la **figure 3** pour ce qui est des paramètres de relaxation. Les activités spontanées sans stimulation électrique (Stim-) ou sous stimulation électrique (Stim+) ont été enregistrées selon la technique De Y Lecarpentier et al. ¹⁰³⁻¹⁰⁵. Les histogrammes pleins correspondent aux matrices collagéniques de base et les histogrammes vides aux matrices collagéniques fonctionnalisées avec les motifs RGD. Les enregistrements ont été effectués au 8^{ème} jour.

Les **figures 2a et 2b** montrent l'effet d'une stimulation électrique sur les matrices. Les paramètres mesurés ont été indifféremment la force développée mesurée en micro newton (cf. « Active tension ») ou les raccourcissements (ΔL). Comme on le voit lorsque la fréquence de stimulation électrique augmente, les

Les figures 2a et 2b montrent l'effet d'une stimulation électrique sur les matrices. Les paramètres mesurés ont été indifféremment la force développée mesurée en micro newton (cf. « Active tension ») ou les raccourcissements (ΔL). Comme on le voit lorsque la fréquence de stimulation électrique augmente, les forces développées diminuent ce qui traduit un effet stercaire négatif. Cependant, quelles que soient les fréquences de stimulation testée, les forces développées en présence du motif RGD sont supérieures aux forces développées en son absence (figure 3a). La force développée est maximum pour une fréquence de 0.17 Hz. En effet, il existe un seuil de stimulation électrique des matrices en fonction de la fréquence. Nous avons encore observé que pour une même intensité de stimulation, le seuil de fréquence de stimulation est plus bas pour les matrices avec RGD. Les réponses mécaniques sont également meilleures en présence du motif RGD (p significatif chacun p<0.05) (figure 2b). Pour les matrices RGD, les seuils de stimulation sont très bas (inférieurs à 3V/cm). Ces seuils sont du même ordre de grandeur que la dépolarisation spontanée mesurable au niveau du myocarde. La capacité du tissu contractile implanté à se synchroniser avec le myocarde natif du patient sera d'autant plus grande que le seuil de stimulation de ce tissu sera bas ce qui est le cas en présence du RGD. Les différents paramètres de contractilité spontanée que nous avons mesurés sont également améliorés dans les matrices RGD + (p<0.01) aussi bien pour ce qui est des paramètres de raccourcissement ((ΔL) (figure 2c) ou pour ce qui est de l'intensité des forces développées (« Active tension ») (figure 2e). De manière intéressante, avec la modification de la matrice les forces mesurées ont été supérieures à toutes celles rapportées dans la littérature. Pour une fréquence optimale de stimulation, la présence du motif RGD entraîne une augmentation d'un facteur 3 à la fois du ΔL (31.1±3.1 vs 9.4±3.9 Δm (cf. RGD- vs RGD+ respectivement, p<0.05). Cette différence persiste même si les matrices sont stimulées (figures 2c, e Stim+ *vs* Stim-). Les figures 2d et 2f rapportent quelques données sur les vitesses de développement de ces forces ou ses raccourcissements en fonction du temps. On voit que aussi bien les vitesses de raccourcissement maximum (maxVc: max vitesse contraction)(figure 2d) et le maximum de force développée dans un temps donné (figure 2f) sont également supérieures dans les matrices RGD. La vitesse maximum de contraction maxVc (633.0± 180.8 vs 56.8±23.1 Am/s (cf. RGD- vs RGD+ respectivement, p<0.05) et la allongements ou de la diminution de la tension musculaire. De plus comme pour les paramètres de contraction la mise en jeu de cette relaxation max Vr : maximum vitesse relaxation et -Df/dt vitesse « diminution de tension active » **(****figure 3d****)** est supérieure dans les matrices modifiées. Cette différence persiste également sous électrostimulation.

Les **figure 3a et 3b** comparent les vitesses de contraction max Vc et de relaxation max Vr da,s les matrices collagénique de base **figure 3a** ou dans les matrices modifiées avec des peptides RGD **figure 3b****.** Pour les deux types de matrices il existe une corrélation linéaire importante entre les vitesses de contraction et de relaxation. Ces vitesses sont par ailleurs pratiquement identiques pour un type de matrice donnée. La contraction est un phénomène actif provoqué par la contraction des cellules. La relaxation est quant à elle un phénomène passif qui peut provenir de la compliance élastique du support matriciel ou un phénomène actif du fait des cellules. Les propriétés mécaniques des matrices de bases non cellularisées RGD + ou moins sont identiques. La différence obtenue en relaxation entre les matrices RGD+ et RGD- ne peut donc pas provenir d'une différence d'élasticité des deux types de matrices mais c'est bien le contingent cellulaire associé à la matrice qui est responsable de la différence observée. Ce qui est également intéressant, étant donné que les vitesses de contraction et relaxation sont très voisines, on en déduit que l'effet matrice est pratiquement nul aussi bien pour ce qui est de l'inertie de la matrice lors de la contraction que d'une éventuelle élasticité lors de la relaxation. Les paramètres de contraction mais également de relaxation sont fondamentaux pour le remplissage myocardique durant la diastole.

L'analyse histologique des différentes matrices confirme une meilleure différenciation des cellules contractiles dans les matrices RGD. Après huit jours, les tissus collagéniques sans RGD ne contiennent pratiquement que des cellules rondes peu différenciées, avec pratiquement aucun contact avec la matrice. Au contraire, les cardiomyocytes associés aux matrices RGD+ sont beaucoup plus alignés, allongés avec un noyau bien régulier et central. De plus, les cardiomyocytes dans les matrices collagéniques (RGD+) ont un arrangement pas du tout anarchique, organisés autour des fibres de collagène avec lesquelles ils adhèrent fortement. L'évaluation du nombre de cellules dans les matrices par FACS montre que le nombre de cellules a diminué en culture. Cependant, cette baisse est d'un facteur 6 après 8 jours dans les matrices collagéniques alors qu'elle n'est que d'un facteur 2 avec les matrices RGD+ (p<0.05 RGD+ versus RGD-). Ainsi, les matrices RGD+ contiennent un nombre plus important de cellules à 8 jours par rapport aux matrices RGD- (0.67 x10⁶ versus 0.20 x10⁶ cellules RGD+ vs RGD- respectivement, p<0.05). De la même façon sur les coupes d'histologie en confocale après marquage du noyau au DAPI, les matrices cellularisées RGD+ ont un nombre plus important de noyaux par section sur les coupes histologiques. De manière très importante, des cross striations transversales (cross striations) des cardiomyocytes qui sont le témoin de l'organisation terminale de l'appareil contractile des cardiomyocytes ont été détectées sur les construits RGD+ mais pas avec les construits RGD-. Les modifications morphologiques des cellules ne sont fonction ni du nombre de cellule initial ni de la densité cellulaire pour une préparation donnée. Ce qui tend a démontrer que les différences observées sont principalement liées à une interaction des cellules avec le support et non pas à une différence d'interaction des cellules entre elles.

Ces résultats démontrent sans équivoque que la fixation de molécules d'adhésion comme les peptides RGD sur le collagène peut améliorer l'adhésion, la survie, et la différenciation de cellules contractiles et ainsi améliorer les propriétés contractiles à la fois spontanées et en cas de stimulation électriques de tissus myocardiques fabriqués à partir de ces supports. Les performances mécaniques sont améliorées mais également les performances électriques. Les tissus sont plus stables électriquement, avec des seuils de stimulation plus bas.

### EXEMPLE 3

Induction d'une angiogénèse in vitro par l'association de cellules endothéliales dans une matrice fonctionnalisée avec des molécules d'adhésion:
Dans un autre type de préparation, des cellules d'intérêt comme des cellules endothéliales (matures ou progénitrices) peuvent être transplantées dans la matrice collagénique 3D fonctionnalisée avec des molécules d'adhésion avantageusement le motif RGD en présence ou non de cellules contractiles ou d'autres types cellulaires comme par exemple des fibroblastes, des kératinocytes, des cellules contractiles, des cellules modifiées génétiquement etc.... qui peuvent être également utilisés indépendamment. Ce support va favoriser la survie et la différenciation de ce contingent cellulaire associé comme le feraient des cellules endothéliales. Ce contingent cellulaire peut être utilisé en association avec des cellules contractiles ou de manière isolée. Le support peut aussi dans certains cas être également utilisé sans contingent cellulaire associé initialement, les cellules colonisant secondairement le support. Il a été cependant démontré que l'association de cellules endothéliales favorise la survie et la différenciation de cellules contractiles implantées dans des supports 3D DA Narmoneva et al. 2004 ³² et que la prévascularisation in vitro de tissu musculaire squelettique favorise sa survie après implantation *(cf. S. Levenberg et al. 2005*) ¹⁰¹.

Les tissus 3D fabriqués in vitro n'ont généralement pas le réseau vasculaire qui existe dans les tissus naturels. Ainsi pour devenir une réalité clinique, un tissu contractile fabriqué in vitro doit être capable de favoriser sa néovascularisation en favorisant le développement d'une vascularisation à partir de la vascularisation préexistante du receveur ou en favorisant la différenciation d'un contingent de cellules endothéliales associées au support 3D initialement ou recrutées secondairement dans ce support après implantation. Ainsi, l'une des propriétés parmi les plus importantes pour un tissu fabriqué in vitro sera sa capacité à favoriser, favoriser sa trophicité et le développement de vaisseaux à l'intérieur afin de limiter au maximum la période ischémique obligatoire qui suit l'implantation et qui est responsable d'une mortalité cellulaire importante avec perte de sa fonctionnalité *(cf. RY Kannan et al (2005)*) ³⁵. Cela devient encore plus critique lorsque l'on s'intéresse à des cellules particulièrement sensibles à l'hypoxie comme des cellules contractiles de type cardiomyocytes et que de plus, ces cellules sont transplantées dans un territoire ou la vascularisation est déjà altérée initialement (myocarde ischémique ou nécrotique). La prévascularisation de l'implant et l'absence de nécessité d'utiliser des gels dans ce type de support permettent de favoriser la libre diffusion des nutriments et donc de fabriquer des tissus d'épaisseurs supérieures. De plus, dans cet espace, le microenvironnement cellulaire peut être contrôlé et modifié de même que la réponse de l'organisme. Possibilité d'associer ce support à des agents ou de transplanter dans ce type de support 3D des cellules transformées avec des gènes codant pour ces agents comme dans le cas présent pour des facteurs de croissance impliqués dans la régulation de l'angiogénèse. D'autres gènes peuvent être bien évidemment associés également et cela de manière non exhaustive comme ceux visant entre autres à favoriser l'adhésion, la survie, la croissance, le « homing », la prolifération, l'apoptose, la différenciation de cellules, à l'intérieur, à proximité ou plus à distance du support 3D. Inclus également des gènes contrôlant la dégradation ou l'élaboration de la matrice extracellulaire, ou des gènes pouvant contrôler la dépolarisation des cellules pour leur conférer une activité rythmique, des gènes codant pour des facteurs régulant la réponse immunitaire, inflammatoire ou tumorale.

### Association du support avec des « Cellules endothéliales »:

A la différence du nombre très important de cellule contractiles nécessaire pour obtenir un tissu contractile de l'ordre de 0.5 à 10. 10⁷ cellules/cm³, le nombre de cellules endothéliales nécessaires est beaucoup plus faible autour de 10⁶ cellules /cm³. Des cellules endothéliales différenciées matures de souris (décrites par Arbiser el al. 1997) ont été transplantées dans la matrice collagénique Mile Sven1 (MS1) (ATCC #CRL-2279). Différentes concentration de cellules MS1 de 10⁴ à 10⁷ ont été mis en place dans les supports collagéniques de type matrice collagénique fonctionnalisée ou pas avec des molécules d'adhésion avantageusement le motif RGD (RGD+ (TR) ou RGD- (T)), mais également des peptides agonistes antagonistes comme le RGE et cultivées en DMEM avec 4 mM L-glutamine, 1.5 g/l sodium bicarbonate, 4.5 g/l glucose, 1 mM sodium pyruvate, penicilline, streptomycine et 5% de sérum de veau foetal (FCS)(Hyclone, Logan, Utah) dans des plaques de culture 12 puits dans un incubateur à 37°C avec une atmosphère de 5% de CO2 pour des périodes de 3 et 6 semaines. Le développement de l'angiogénèse in vitro a été étudiée en microscopie électronique. Les formations vasculaires ne sont pas visibles aux faibles grossissements obtenus avec les microscopes classiques, et la présence de structures vasculaires doit être démontrée à plus fort grossissement en ayant recours à la microscopie électronique (EM).

Pour la microscopie électronique, les préparations ont été fixées dans paraformaldéhyde, 1.5% glutaraldéhyde et 1 mM calcium in 0.1M tampon sodium cacodylate (pH 7.2) à 4°C puis fixés dans un tampon avec 1% OsO₄ à 4°C et fixés en bloc avec 1% uranyl acétate dans 50% éthanol avant d'être incorporés dans des résines d'Epon/araldite (Electron Microscopy Sciences, Fort Washington PA). Des sections plus fines (60nm) ont été réalisées et colorées avec de l'unranyl acétate et citrate. Tous les prélèvements ont été examinés en utilisant un microscope électronique CM-100. La recherche de formation vasculaire à été effectuée en microscopie avec un grossissement de (4k) et les structures vasculaires ont été confirmée grâce à une analyse à plus fort grossissement pour identifier les jonctions serrées et la présence de membrane basale en faisant varier l'incidence du rayonnement Rx si besoin. Une lumière vasculaire a été définie comme un espace fermé délimité par des cellules endothéliales interagissant par des jonctions cytoplasmiques élaborées de type « tight junction ». Le nombre de structure vasculaire par mm² a été quantifié, le degré de complexité des vaisseaux a été évalué par la mesure du rapport nucléocytoplasmique des cellules impliquées, par le nombre moyen de cellules participant à la formation d'une lumière et par le % de ramification défini par le nombre de lumières en contiguïté et le nombre moyen de structure vasculaire en cas de ramification. L'analyse statistique a fait appel aux tests pour séries appareillées type Wilcoxon Matched-Pairs Signed-RanksTest.

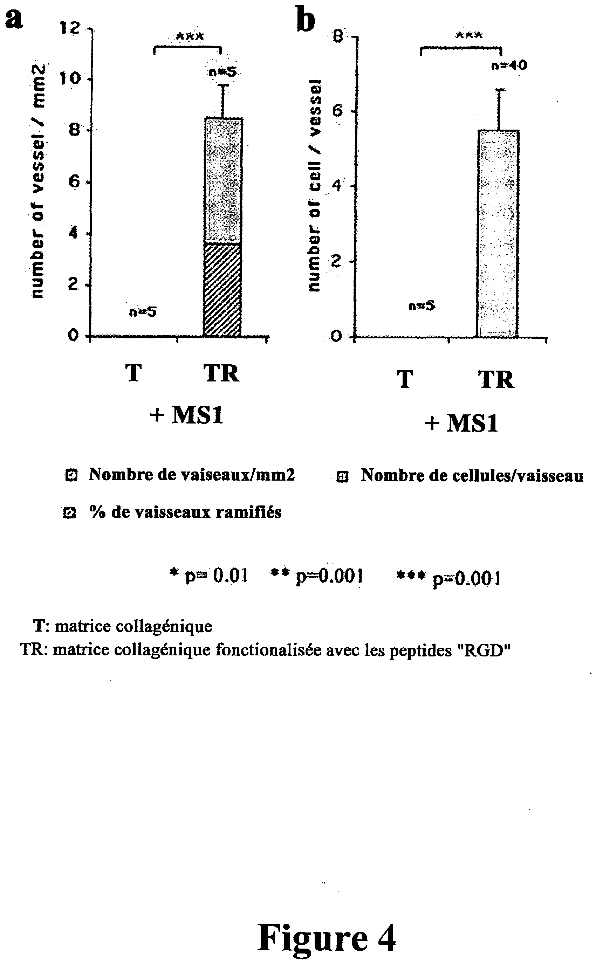

Dans la matrice collagénique fonctionnalisée avec les peptides RGD, les cellules ont modifié leur aspect morphologique en devenant beaucoup plus étalées à 3 et 6 semaines. Une interaction des cellules avec la matrice par le biais de molécule d'adhésion est responsable de cette modification morphologique de la cellule puisque cet modification de l'aspect n'est pas fonction du nombre initial de cellules et n'est pas présent dans les matrices collagéniques de bases ou dans les matrices fonctionnalisées avec le RGE. De plus cette modification disparaît si la forme soluble du motif RGD est associée au milieu de culture. Dans la matrice RGD, les cellules endothéliales sont polarisées. Il existe d'autre part, à certains endroits une membrane basale entre les cellules et la matrice. Cette membrane basale n'est pas présente si les cellules sont cultivées dans les matrices collagéniques non fonctionnalisées. Alors que l'obtention de structures de type vasculaire in vitro en microscopie classique (« capillary-like network ») dans les matrices collagéniques nécessite classiquement la présence d'un agent de différenciation comme le PMA (cf. Ilan, 1998) ³⁶ ou la présence de fibroblastes (cf. Black, 1998) ¹⁰⁶ ou un milieu enrichi par des facteurs sécrétés par des fibroblastes ((cf. Montesano, 1993 ; Baatout, 1997) ^{107,108}, de véritables structures vasculaires se développent spontanément dans les matrices RGD comme observé en microscopie électronique avec une moyenne de 8.5 « structures vasculaires » par mm² +/- 1.3 à 6 semaines (n=5 expériences séparées) **(****figure 4a****).** Dans le même temps aucune de ces structures n'est présente dans les autres types de matrice **(****figure 4a****).** La plupart des « lumières vasculaires » sont formées par les prolongements cytoplasmiques et non pas par le corps cellulaire qui contient le noyau. Une étude attentive de plus de 40 structures vasculaires dans les matrices RGD+ montre que le nombre moyen de cellules impliquées dans la formation d'une lumière vasculaire était de 5.5+-1.1 (cf. fig. 4b). Dans certains cas, les lumières vasculaires sont formées par plus de 10 cellules endothéliales interconnectées. 23.6% des vaisseaux sont considérés comme ramifiés (**figure 4a****,** partie hachurée) avec un nombre de lumières vasculaires de 3 +/0.5 en cas de ramification.

### EXEMPLE 4

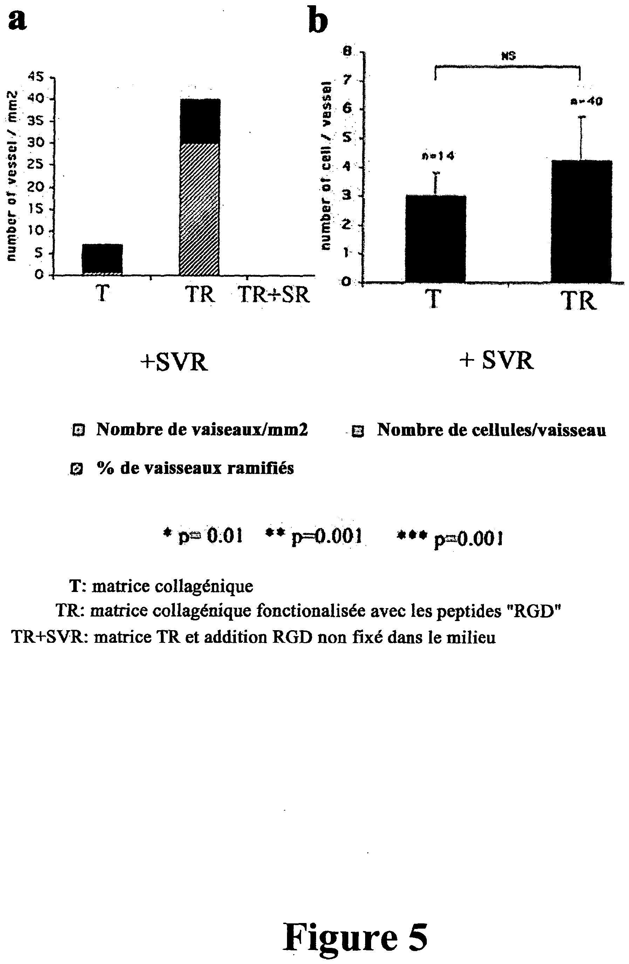

Possibilité d'implanter des cellules modifiées génétiquement qui sont capables de sécréter un agent dans le support collagénique fonctionnalisé :
Dans l'exemple suivant les cellules transplantées dans la matrice peuvent être modifiées génétiquement pour libérer un agent biologique impliqué par exemple dans l'angiogénèse, la migration, la survie, la prolifération, l'apoptose, la différentiation, le contrôle de la dégradation, de l'élaboration de la matrice extracellulaire, le contrôle de la réponse immunitaire, inflammatoire ou tumorale etc.... Il a été montré qu'il était possible de modifier génétiquement des cellules contractiles comme des myoblastes avec des gène impliqués dans l'angiogénèse comme VEGF, angiopoïtine etc... afin d'obtenir des tissus de meilleure qualité après transplantation (*D.E. Coppi et al. (2005)*) ¹⁰⁹. Dans le cas présent, les cellules de type endothélial (cf. MS1) ont été modifiées par transfection virale avec le gène d'activation Ras+. La voie Ras/Map Kinase a été rapportée comme une voie d'activation très importante dans le contrôle de l'angiogénèse (cf. Ilan et al. 1998) ³⁶. Les cellules MS1 Ras+ sont connues sous le nom de SEVN 1 ras (SVR) (ATCC #CRL-2280) (cf. Arbiser, 1997) ¹¹⁰. Les cellules SVR **(****figure 5****)** ont été implantées et cultivées dans les différentes matrices comme précédemment pendant 6 semaines. L'exemple montre que l'activité biologique des cellules peut être modifiée par manipulation génétique et qu'il reste cependant possible en fonctionnalisant les matrices avec des molécules d'adhésion, d'influencer leur comportement et leur degré de différenciation. La transformation de cellules endothéliales avec Ras augmente leur potentiel angiogénique puisque des structures vasculaires et la présence de membranes basales sont visibles même dans les matrices collagéniques de bases (T) avec environ 7 structures vasculaire/mm2 **(****figure 5a****).** Mais même avec ce type de cellules, l'angiogénèse reste beaucoup plus développée si ces cellules sont mises en place dans des matrices fonctionnalisées avec des molécules d'adhésions (TR) **(****figure 5b****).** La présence des molécules d'adhésion permet d'obtenir une angiogénèse plus développée à la fois en quantité (nombre de structures vasculaires/mm2 ((40 vs 7) (RGD+ *vs* RGD-) (p significatif)) **(****figure 5a**) mais aussi en qualité (% de vaisseaux ramifiés ((75% *vs* 10%) (RGD+ vs RGD-)(p significatif))**(****figure 5a**), niveau de complexité des vaisseaux avec notamment le rapport nucléocytoplasmique plus faible et un nombre de cellules impliquées dans chaque lumière vasculaire plus élevé ((4.5 vs 3)(RGD+ *vs* RGD-)((ns)) (cf. **figure 5b**).

### EXEMPLE 5

Evaluation de l'intérêt de la fixation covalente de la molécule d'adhésion sur le support collagénique :
Pour étudier l'importance de la fixation au support dans l'effet observé, la forme soluble du motif RGD ou la simple adsorption du motif RGD ont été testées en présence de cellules contractiles ou de cellules endothéliales dans des supports collagéniques. De fortes concentrations de RGD de 1 à 1000 µg/ml ont été associées au milieu de culture ou adsorbées sur la matrice pendant plus de 24h avant l'implantation des différents types de cellules. Dans tous les cas, l'effet bénéfique du motif RGD sur la différenciation et la survie des cellules endothéliales ou des cardiomyocytes est évident. Qui plus est, même si les cellules sont cultivées dans un support collagénique modifié avec le peptide d'adhésion, la présence de la forme soluble du motif RGD dans le milieu inhibe cet effet **(****figure 5a**).

### EXEMPLE 6

Utilisation de la matrice fonctionnalisée avec des molécules d'adhésion en présence d'un agent biologique.

Certains de ces agents comme par exemple des facteurs de croissance peuvent être simplement associés au milieu de culture in vitro, adsorbés sur le support collagénique *(cf. EJ Suuronen et al. (2003)) ⁵⁴* ou encore fixés de manière covalente en utilisant ou non le moyen que nous avons proposé. La matrice peut être également fonctionnalisée avec des protéoglycanes par exemple qui ont la propriété de retenir les facteurs de croissance. Certains de ces agents peuvent être par exemple des facteurs de croissances, des agents stabilisant les facteurs de croissance (comme par exemple "oligomeric regenerating agents » (RGTAs)), des chémokines, des agents visant à augmenter la contractilité, des agents augmentant l'angiogénèse, contrôlant la réaction inflammatoire, contrôlant sans dégradation ou favorisant sa polymérisation etc.. L'agent peut être présent dans le support ou administré à la périphérie du support ou dans un dispositif associé visant par exemple à contrôler le remodelage ventriculaire, ou traiter un myocarde pathologique. Le support peut également servir pour délivrer ou concentrer, ou maintenir ces agents dans un endroit donné. L'agent peut être également produit par les cellules associées au support naturellement ou après modification. Les facteurs de croissance sont classiquement utilisés à des concentrations de 1 fg/ml à 1 mg/ml (cf. 1-10 nM) (*cf. EJ Suuronen et al. (2003))⁵⁴* mais des concentrations différentes peuvent être utilisées.

Dans cet exemple, l'agent associé est un facteur de croissance visant à augmenter l'angiogénèse comme le classique VEGF. Le facteur de croissance a été associé au milieu de culture, mais ce facteur peut être également simplement adsorbé sur le collagène pendant quelques heures avant la mise en place des cellules ou fixé par son site NH2 par exemple.

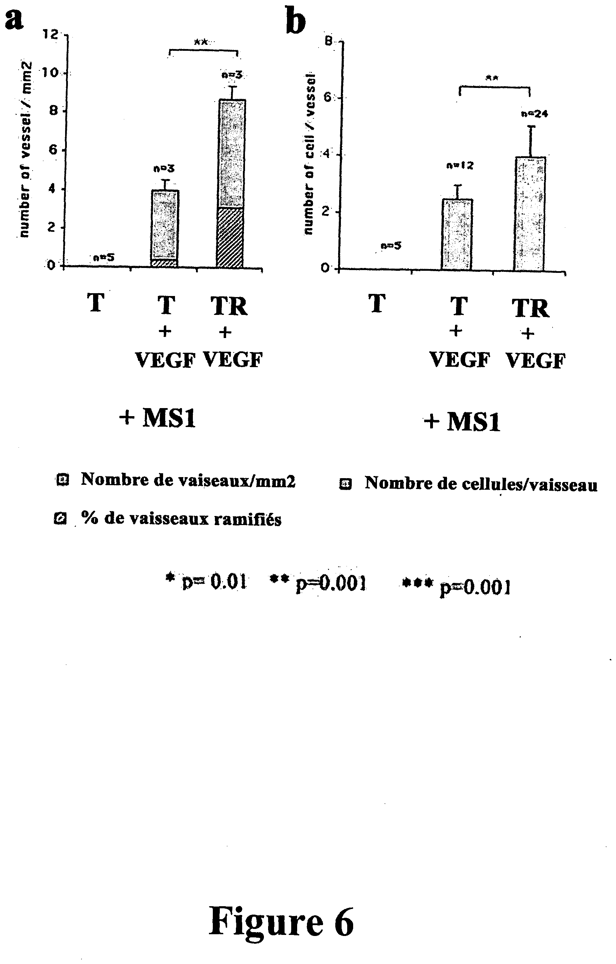

La présence de VEGF (VEGF ₁₆₄ (R∝D, Minneapolis, MN) à des concentration de 4 ng/ml induit la différentiation des cellules MS1 avec l'apparition de structures vasculaires dans les matrices de collagène de base (T) et cela même en l'absence de fonctionnalisation avec des molécules d'adhésions **(****figure 6a****).** Le nombre absolu de ces structures reste cependant faible (en moyenne 3/mm2), de même que leur niveau de complexité : uniquement 2.5 +/-0.5 cellules (n=12) par lumière vasculaire **(****figure 6b****),** avec des vaisseaux aux parois épaisses (pratiquement pas de prolongement cytoplasmique), seulement 10% des structures sont ramifiées **(****figure 6a****)** avec en cas de ramification uniquement 2 lumières vasculaires en continuité seulement. Il est connu que les vaisseaux obtenus avec VEGF ne sont pas parfaitement formés et ont tendance à favoriser des extravasations vasculaires. En présence du motif RGD (TR) par contre, la densité des structures vasculaires augmente 8.7+-0.7/mm2 **(****figure 6a****),** de même que leur niveau de complexité : nombre de cellule par lumière vasculaire 4 +/-1.1, 25% de ramifications, 3.5 lumières en contiguïté en cas de ramification)**(figure 6a-b)****.**

### EXEMPLE 7

Les supports collagéniques fonctionnalisés avec des molécules d'adhésion comme les peptides RGD ont un effet anti-apoptotique sur les cellules qui leur sont associées

Les récepteurs intégriniques, en plus de leur rôle dans l'adhésion des cellules à leur environnement jouent un rôle clef en limitant la mort cellulaire par apoptose et en augmentant les gènes de survie (*Meredith et al. 1997*) ¹⁸. En fait la plupart des cellules adhérentes comme les cardiomyocytes, lorsqu'elles perdent le contact avec la matrice environnante deviennent apoptotiques (*D. Kuppuswamy et al. (2002)*) ¹⁷. Une partie de la mort cellulaire pourrait être due à l'inhibition de cette interaction. Cela est probablement une des raisons pour laquelle l'injection de cellules seules dans les tissus est peu efficace et accompagnée d'une mortalité très importante de l'ordre de 95% ce qui justifie l'utilisation d'une matrice 3D au moment de l'injection pour recréer cet environnement. Il faut cependant que le support 3D possède également les propriétés mécaniques et les ligands spécifiques nécessaires à cette interaction dans le système 3D.

L'apoptose a été étudiée in situ en utilisant un microscope confocal type Zeiss avec un logiciel pour intégrer les données (MRC 1024, Biorad). Les membranes des cellules dans la matrice ont été marquées in situ en utilisant une coloration Dye red. Les cellules apoptotiques ont été marquées en utilisant un marquage fluorescent vert anti-annexine V (FITC). Une quantification de l'apoptose a été réalisée en comparant la surface de fluorescence rouge correspondant à l'ensemble des cellules à la fluorescence verte correspondant aux cellules apoptotiques. membranes des cellules dans la matrice ont été marquées in situ en utilisant une coloration Dye red. Les cellules apoptotiques ont été marquées en utilisant un marquage fluorescent vert anti-annexine V (FITC). Une quantification de l'apoptose a été réalisée en comparant la surface de fluorescence rouge correspondant à l'ensemble des cellules à la fluorescence verte correspondant aux cellules apoptotiques.

Après 3 semaines, le pourcentage de cellules MS1 apoptotiques dans les matrices fonctionnalisées avec le motif RGD est de 10% contre plus de 80% en l'absence de ce motif. De la même manière que ce que nous avons rapportés pour la différenciation cellulaire, la présence de la forme soluble du motif RGD dans le milieu annule cet effet et dans les deux types de matrices fonctionnalisées ou pas avec le motif RGD, l'apoptose revient à sa valeur de 80%. D'où la nécessité que le motif soit fixé. La faible survie de cellules endothéliales dans différents types de support 3D collagéniques (*cf. Ilan et al.,* 1998, *Satake et al.,* 1998, *Goto et al., 1993*)^{36,37,39} ou type Matrigel (*cf. Ranta et al. 1998*)³⁸ a déjà été rapportée par différents groupes. La présence d'un motif RGD fixé serait un moyen de diminuer cette mort cellulaire dans ce type de support et d'améliorer l'efficacité de ces transplantations cellulaire après injection.

### EXEMPLE 8

Contrôle de la biodégradation du support collagénique par un traitement non cytotoxique compatible avec la thérapie cellulaire : fixation par le glutaraldéhyde associée à l'utilisation d'un protéoglycane comme l'héparine ou à l'utilisation de génipine :
La méthode la plus efficace pour fixer le collagène reste une réticulation chimique par le glutaraldéhyde. Cependant le glutaraldéhyde peut se polymériser et se dépolymériser lentement en libérant du glutaraldéhyde libre qui est toxique pour la cellule.

### Fixation du tissu avec glutaraldéhyde suivi par un traitement au chitosan et à l'héparine modifiée (matrices collagène-glut / GAG)

La fixation par le glutaraldéhyde est faite par incubation des supports collagéniques Ultrafoam@ dans un tampon NaCl (50 mM) contenant 0.625 % de glutaraldéhyde pendant un mois à 37°C. Après lavage, les supports collagéniques sont placés dans une solution à 0.2% de chitosan (Sigma Aldrich) puis 1% glycine (Sigma Aldrich) et de sulfate de gentamicine 0.02% (Sigma Aldrich) pendant deux semaines à 20 °C. Une solution d'héparine partiellement dégradée est préparée par action de nitrite de sodium en présence d'acide chlorhydrique (1M, pH 2.0) à 4°C 3h sur du sulfate d'héparine. La solution est ensuite amenée à pH 7.4 par de la soude N et les supports de collagène sont mis en contact avec la solution pendant 12 h. Les préparations de collagène sont ensuite lavées avec un tampon NaCl (50 mM) puis mises en contact 6h à 20 °C avec une solution de cyanoborohydrure de sodium (Sigma Aldrich) à 1% dans un tampon NaCl (50 mM) préparée la veille. Les préparations de collagène sont ensuite à nouveau lavées et maintenues dans une solution contenant 1% glycine et 0.02% de sulfate de gentamicine jusqu'à leur utilisation finale.

### Fixation par la génipine (matrices collagène / génipine):

L'utilisation de génipine pour fixer du tissu frais ou decellularisé a déjà été proposés (US patent Acellular biological material chemically treated with genipin Sung Hsing-Wen et al. 2003). Nous avons suivi le mode opératoire proposé par le auteurs

La génipine (Challenge Bioproducts CO., Taiwan) a été utilisée à 0.625% en PBS pH 7.4 à 37°C pendant 3 jours

### Evaluation in vivo de matrices collagéniques traitées avec les procédés précédents :

Différents types de matrices ont été implantés au niveau des muscles spinaux de rats sous anesthésie. Les analyses histologiques après 10 jours ont mis en évidence une infiltration massive des matrices collagénique de base par des cellules inflammatoires avec une dégradation intense et précoce de la trame collagénique et remplacement de cette matrice par de la fibrose peu vascularisée.

Dans les matrices collagène-glut / GAG tout comme dans les matrices collagène / génipine l'infiltration cellulaire est limitée à la périphérie de la matrice. De manière intéressante, ces matrices favorisent le développement d'une angiogénèse importante au voisinage de la matrice bien que la pénétration des vaisseaux à l'intérieur de la matrice reste limitée. La présence de protéoglycanes dans la matrice qui sont connus pour favoriser l'adsorption et la présentation de facteurs de croissance pourrait expliquer en partie l'augmentation de l'angiogénèse locale. Après 1 mois toutes les matrices collagéniques 16/16 ont disparu mêmes celles dont le diamètre initial était le plus important (8 mm). Au contraire, toutes les matrices collagène-glut / GAG, n=16 ou collagène / génipine, n=16 présentent une dimension inchangée (p significatif) et l'infiltration cellulaire reste limitée à la périphérie. D'autre part, les cellules inflammatoires sont peu représentées dans cette infiltration.

### EXEMPLE 9

Fonctionnalisation de support collagène-glutaraldéhyde/GAG ou collagène/ génipine par la fixation de molécule d'adhésion :
Cette association permet de contrôler la réponse inflammatoire ou immunitaire vis-à-vis d'un tissu et de sélectionner sa colonisation par des cellules d'intérêts.

La possibilité de modifier les matrices avec des peptides d'adhésion a été également validée. ces molécules d'adhésion pouvant être associées avant ou après les agents de réticulation du collagène. Les peptides d'adhésions comme le RGD ont été fixés sur les matrices de collagène Ultrafoam® en utilisant le mode opératoire décrit dans l'exemple 1 puis les matrices ont été fixées ensuite suivant les méthodes décrites dans l'exemple 8. Après rinçage en PBS, ces matrices ont été implantées dans les muscles spinaux de rat sous anesthésie. Jusqu'à 8 matrices ont pu ainsi être implantées chez le même animal.

Après 10 jours et 1 mois d'implantation, toutes les matrices glut/GAG sont encore présentes avec peu de résorption tandis qu'en absence de traitement par glutaraldéhyde, toutes les matrices collagéniques fonctionnalisées ou pas avec le motif RGD ont déjà disparu. Les analyses histologiques après 10 jours ont mis en évidence une infiltration massive des matrices collagénique de base par des cellules inflammatoires avec une dégradation intense et précoce de la trame collagénique et remplacement de cette matrice par de la fibrose peu vascularisée. Le traitement par glut /GAG limite l'infiltration cellulaire initiale de la matrice par des cellules inflammatoires et la dégradation de la matrice est retardée avec une angiogénèse qui reste limitée à la périphérie. De manière fort intéressante, en présence de motifs d'adhésion comme les motifs RGD (matrices collagénique-glut/PGA), les vaisseaux pénètrent cette fois à l'intérieur de la matrice de même que les cellules du tissus environnant et même très rapidement puisque après seulement 10 j. d'implantation, les 2/3 externes de la matrice sont déjà colonisés et vascularisés. Une analyse de la cinétique de l'angiogénèse dans les matrices grâce à la technique de quantification de l'angiogénèse en utilisant l'injection intravasculaire d'un marqueur fluorescent ISL-B4 (Molecular Probes) confirme que les matrices deviennent vascularisées et que cette angiogénèse commence dès la deuxième semaine pour atteindre un plateau aux alentours de 6% après 3 semaines. D'autres expériences pour des périodes de temps plus longues ont confirmé que cette angiogénèse n'augmente que lentement mais que, contrairement à ce qui a lieu avec d'autre types de support comme les matrices Matrigel™, cette angiogénèse n'a pas tendance à diminuer après quelques mois. A noter que la quantité d'angiogénèse dans les matrices doit être comparée à celle observée dans les muscles environnants comme les muscles spinaux ou cette angiogénèse est de l'ordre de 3%+/-1%.

Il existe une colonisation cellulaire de la matrice qui n'est pas spécialement le fait de cellules inflammatoire. Cette approche est très intéressante car elle propose un premier traitement qui vise à empêcher la colonisation cellulaire d'un support 3D puis de sélectionner par le biais de molécule d'adhésion choisies des cellules d'intérêt comme, par exemple, des cellules endothéliales. La présence d'une néoangiogénèse intense dans les matrices collagéne-glut./GAG modifiées avec les peptides d'adhésion montre une très bonne biocompatibilité de ce type d'implant et plaide pour une faible toxicité compatible avec son utilisation en thérapie cellulaire.

### EXEMPLE 10

### Utilisation de supports collagéniques réticulés et fonctionnalisés ou pas avec des molécules d'adhésions comme les peptides RGD pour la thérapie cellulaire:

La capacité de cellules transplantées à survivre et à se différencier dans ce type de supports a été analysée. Des cellules humaines ou pas plus ou moins différenciées: cellules souches, cellules progénitrices, cellules matures, cellules modifiées ou pas génétiquement ont ainsi été implantées dans les différents types de support fonctionnalisés ou pas avec les peptides RGD. La participation de ces cellules dans l'angiogénèse des supports a été évaluée dans un modèle d'implantation de support dans des tissus musculaires (type muscles squelettiques) et une méthode d'analyse quantitative de l'angiogénèse dans ces supports a été développée.

### Cellules :

- Des cellules endothéliales matures de souris MS1.- Des cellules endothéliales matures de souris modifiées génétiquement SVR .
- Des cellules souches de souris isolées à partir de tissus différenciées. Nous avons récemment rapportées l'isolation et la caractérisation de progéniteurs pour la cellule endothéliale isolés dans des tissus différenciés pendant le développement de la souris (*cf S. Cherqui, SM. Kurian, O. Schussler et al. (2006*)) ¹¹¹. Ces progéniteurs ont été isolés à partir de la fraction de cellules ayant une faible capacité d'adhésion à partir d'extraits de foie de souris néonataux digérés enzymatiquement par de la collagénase. Les cellules ont été collectées au 8^{ème} jour de culture in vitro.
- Des cellules endothéliales matures humaine isolée à partir de la paroi des veines de cordon connues sous le nom de « HUVEC » .
- Des progéniteurs d'origine humaine pour la cellule endothéliale isolés à partir du sang du cordon ombilicale. Ces cellules ont été préparées comme cela a été décrit précédemment (*cf. Crisa et al. (1999)* and Hildbrand et al. (2004) ^{112,113}.

### Modèle d'angiogénèse in vivo:

Les différents types de cellules endothéliales ont été mis en place dans les matrices traitées avec glutaraldéhyde/GAG et modifiées ou pas avec des peptides d'adhésion comme les peptides RGD. Des souris immunodéprimées ont été anesthésiées au pentobarbital et les matrices ont été implantées dans leurs muscles spinaux pour des périodes allant jusqu'à 6 semaines. Huit matrices ont pu ainsi être implantées par souris. Différentes combinaisons ont pu être ainsi testées chez le même animal. Les matrices ont été explantées après différents délais et l'angiogénèse dans les matrices a été quantifiée.

### Quantification de l'angiogénèse in vivo:

Nous avons développé une technique fiable et reproductible pour la quantification de l'angiogénèse in vivo. Les vaisseaux ont été marqués in vivo avec un marqueur fluorescent anti cellule endothéliale de souris MECA32 (Université de lowa, US) ou ISL-B4 (Vector Laboratories, Burlingame, CA). Après sections épaisses (200 µm) en utilisant un couteau oscillant «Automatic Oscillating Tissue Slicer » (OTS-4000) (Electron Microscopy Sciences) nous avons utilisés la transillumination à travers toute l'épaisseur de la matrice pour évaluer l'angiogénèse dans un volume de tissu donné. La fluorescence à alors été étudiée en utilisant un microscope à fluorescence classique et l'intensité de la fluorescence a été digitalisée. En fonction de l'intensité de la fluorescence les vaisseaux ont pu ainsi être reconnus. La variabilité intra ou inter observateur variabilité est de moins de 10 % pour un spécimen donné. Les comparaisons statistiques ont été effectuées en utilisant des tests type ANOVA ou des tests t de Student.

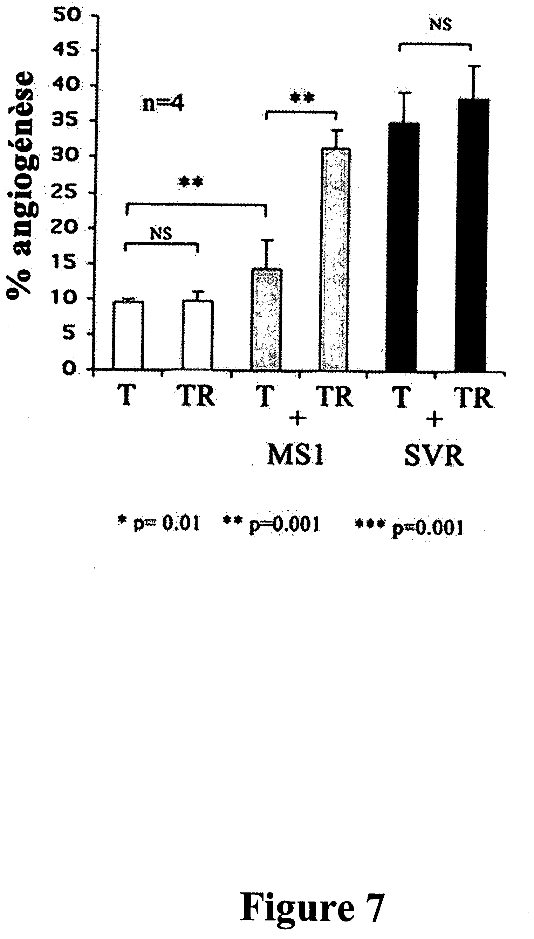

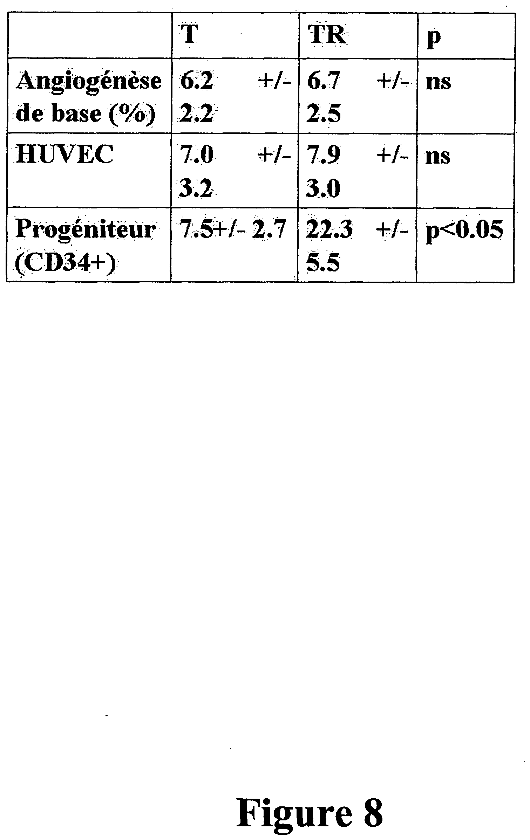

En l'absence de contingent cellulaire associé, l'angiogénèse dans la matrice collagénique reste faible autour de 8% (**figure 7** (T ou TR)). La présence de cellule endothéliale type MS1 augmente l'angiogénèse dans la matrice collagénique aux alentours de 14% **(****figure 7****)** (T+MS1)). Cette augmentation est encore plus marquée si la matrice est fonctionnalisée avec les peptides RGD 35% (p significatif)(cf. fig. 7 (TR+MS1)). En présence de la forme transformée de MS1 (cf. SVR), l'angiogénèse est déjà très importante dans les deux types de matrices autour de 40%. Les cellules de la moelle hematopoïetiques ou mésenchymales, les leucocytes isolés à partir du sang de la veine ombilicale ou du sang périphérique, les cellules souches, les cellules amniotiques, les cellules epiploïques, les cellules dérivées du tissus adipeux etc..... toutes ces cellules peuvent dans certaines conditions participer à l'angiogénèse et donc être intéressantes à associer à d'autres contingents cellulaires. Dans les expériences de Crisa L. et al. Les cellules endothéliales humaines type HUVEC ne formaient que très peu de vaisseaux dans des matrices collagéniques *(cf. Crisa et al. (1999)¹¹³*. En accord avec cette observation, dans notre expérience, la quantité d'angiogénèse dans des matrices modifiées avec le peptide RGD (T ou TR) avec ou sans cellule endothéliale humaine (HUVEC) était sensiblement identique (6.7% +/- 2.5 *vs* 7.9% +/- 3 *ns*) **(****figure 8****).** De manière fort intéressante, l'angiogénèse dans les matrices collagéniques peut être augmentée en présence de progéniteurs pour la cellules endothéliale isolés à partir du sang circulant (cf. progéniteurs CD34+) jusqu'à (22.3% +/- 5.5) mais seulement si les matrices ont été modifiées avec le peptide RGD **(****figure 8****).** Cela démontre donc clairement l'intérêt d'utiliser les motifs d'adhésions dans les matrices collagéniques avec des cellules humaines.

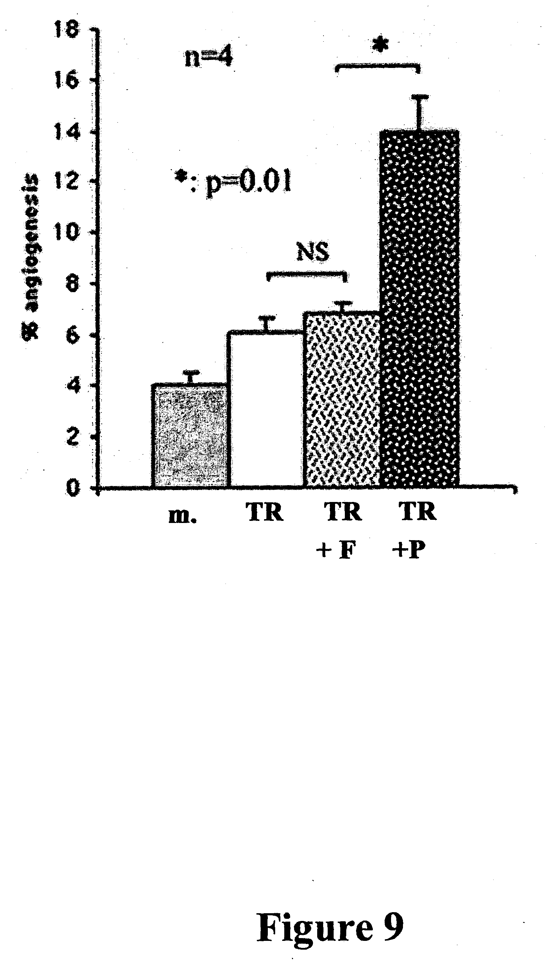

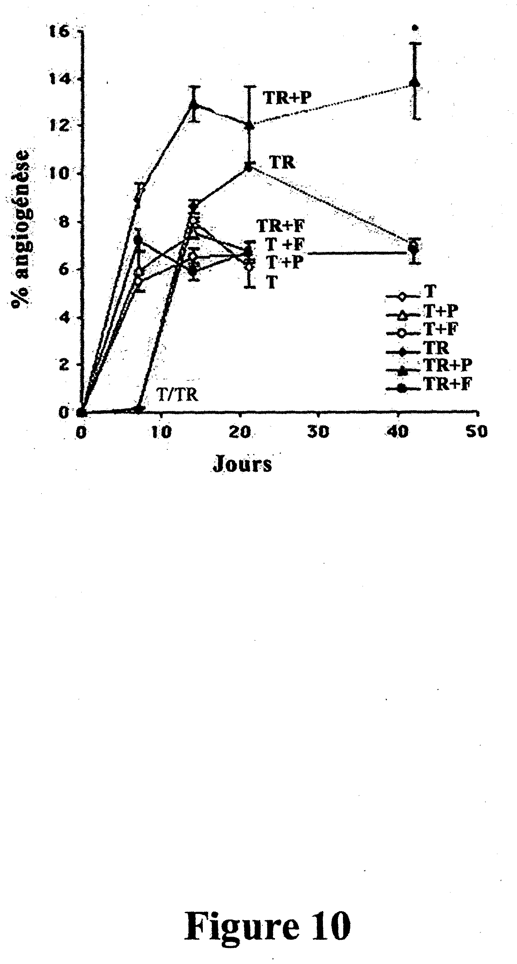

Différentes approches sont en cours de développement pour augmenter l'angiogénèse dans des environnements tridimensionnels. Les préparations à partir de tissus foetaux peuvent également déjà contenir ces cellules et la présence du motif RGD pourrait favoriser la survie et la différenciation de ces cellules. Nous avons récemment rapportés la présence dans les tissus foetaux différenciés de cellules souches qui sont capable de s'incorporer dans l'architecture vasculaire de l'hôte pour en augmenter l'angiogénèse locale (*cf. S. Cherqui et al. (2006*) ¹¹¹. Ces cellules sont très dépendantes pour leur survie de facteurs sécrétés par les cellules « feeder » essentiellement des fibroblastes (F). A 3 semaines, le potentiel angiogénique de ces cellules feeder « F » reste faible si on les compare à celui des progéniteurs pour la cellule endothéliale « P » et l'angiogénèse obtenue est du même ordre de grandeur que celle obtenue dans la matrice sans cellule ou dans les muscles spinaux environnants **(****figure 9****).** Cependant, comme cela est montré sur la **figure 10****,** la présence d'un contingent cellulaire accélère l'angiogénèse initiale qui est alors de 6% à 7 jours alors qu'elle n'est pas encore détectable en l'absence de cellule associée. Plus tard, la quantité d'angiogénèse est augmentée dans les matrices modifiées avec le peptide RGD, mais seulement en présence du progéniteur pour la cellule endothéliale pour atteindre un plateau autour de 14% alors que en l'absence de RGD ou en présence de cellules nourricières (« feeder cell »), l'angiogénèse reste basse et stable **(****figure 10****)**

### Applications

Cette approche pourrait donc avoir un intérêt double, le premier serait de favoriser et d'accélérer l'angiogénèse initiale en ayant recours à des cellules qui ont une capacité proangiogénique et le deuxième serait d'associer au tissus lui-même un contingent de cellules endothéliales ayant la capacité de se différencier et de former des vaisseaux dans un environnement 3D modifié qui le lui permette. Autant de paramètres qui vont limiter les phénomènes ischémiques initiaux post implantation et augmenter l'angiogénèse en territoires ischémiques ou nécrosés.

Les matrices ont été implantées dans les muscles spinaux parce que, comme pour le tissu myocardique, il s'agit d'un tissus musculaire et d'autre part dans certains cas, avant d'être implanté au contact du myocarde, le tissu cellularisé peut aussi être implanté dans des sites ectopiques bien vascularisés, le patient receveur servant en fait de bioréacteur pour développer son propre tissu. Ces sites pourraient être par exemple la cavité péritonéale avec notamment l'épiploon, un tissu musculaire comme le grand dorsal par exemple, la cavité pleurale, tissu sous cutané etc..... Dans ces sites, différents échafaudages pourraient être accumulés les uns sur les autres de manière à ce qu'un tissu d'épaisseur supérieure bien vascularisé puisse être obtenu ¹¹⁴. Dans ces différents sites différents stimuli pourraient être appliqués (chimiques, biologiques, physiques etc). Ces stimuli pourraient être entre autre une stimulation électrique qui ont montré leur intérêt pour améliorer la qualité d'un tissu contractile, un stress mécanique. Les différents stimuli pourraient ou non être synchronisés sur la fréquence cardiaque du patient. Un moyen pour appliquer le « shear » stress serait de fixer l'échafaudage à un support dont le volume peut varier comme un système gonflable comme un ballon ou une bouée qui pourrait avoir par exemple la forme des cavités ventriculaires. Le support pourrait être en silicone par exemple afin de limiter l'adhésion de la matrice à ce support. Une partie de l'échafaudage pourrait être couvert par des tissus ou des dispositifs qui visent à limiter les adhésions locales afin de faciliter les réinterventions itératives. Après plusieurs semaines, l'échafaudage vascularisé pourrait être alors transféré dans le péricarde pour être appliqué sur le coeur pour remplacer une partie ou la totalité de la paroi myocardique, pour traiter des perturbation du fonctionnement myocardique, en tant que greffon libre ou greffon pédiculisé³⁰. Dans certains cas, un lambeau epiploïque ou de muscle squelettique par exemple, un lambeau pourrait être utilisé soit pour couvrir le patch contractile soit pour transférer le patch dans le péricarde. L'utilisation de patch contractile peut être également associée à l'utilisation d'autres méthodes de régénération myocardique, comme la thérapie cellulaire. Dans cette invention nous proposons l'utilisation de « fibrines modifiées » dans les différentes applications médicales et chirurgicales ou la fibrine est classiquement employée. Une des applications étant l'ingénierie de tissu, la thérapie cellulaire, ou l'amélioration de dispositifs médicaux/chirurgicaux où des supports contenant de la fibrine/fibrinogène sont utilisés.

L'utilisation de supports injectables comme par exemple des supports à base de collagène (micro ou nanoparticules de collagènes associée ou non avec d'autres composants pour favoriser leur polymérisation ou association à un contingent cellulaire modifiés pour sécréter des facteurs ou activité par exemple enzymatique visant à provoquer la polymérisation du collagène) ou fibrine injectable modifiée ou pas avec des molécules d'adhésions (un gel à base de fibrine ou de collagène avec une modification du fibrinogène ou de la fibrine avec des motifs d'adhésions) associés ou pas à des cellules pourrait être utiliser pour faciliter l'attachement du tissus contractile au myocarde ou par exemple afin de préparer ou d'aider la préparation du site ou va se faire le transfert du patch contractile en le prévascularisant par exemple. Les thérapies cellulaires pourraient améliorer les résultats obtenus avec la simple utilisation de patch contractile. Les supports injectables pourraient,être injectés dans un tissu, dans un support, dans un dispositif utilisé en cardio-thoracique et vasculaire, dans un vaisseau ou combinaison. Les cellules pouvant être associé initialement, en même temps ou secondairement ou combinaison. Tous les différents types cellulaires ne sont pas nécessairement présent dans le support au même moment. Le support injectable peut être utilisé indépendamment du patch.

Dans certaines formes, l'échafaudage pourrait être associé à un dispositif qui viserait à prévenir les remodelages ou dilatation ventriculaire comme des filets par exemple ou un dispositif qui viserait à traiter des perturbations myocardiques. Ces dispositifs pourraient entourer une partie ou tout le coeur. Ce dispositif pourrait être dans certains cas essentiellement composé par la matrice contractile. L'application d'un échafaudage externe pourrait être associé à l'injection de cellules dans le myocarde. Ces cellules pourraient être injectées dans l'échafaudage 3D modifié avec les molécules d'adhésions comme le motif RGD.

Afin de favoriser la revascularisation de ces supports, des chemokines qui favorisent le recrutement des cellules souches pourraient être utilisées comme SDF1, SCF, TGF-alpha, FGF etc.... Des agents visant à mobiliser les cellules souches du patient pourraient être également utilisés afin de favoriser la revascularisation de l'implant.

Nous conduisons actuellement une étude multicentrique internationale chez l'homme en comparant l'injection simple intra myocardique de cellules de la moelle hématopoïetique dans des zones infarcies à cette même injection de cellules mais cette fois associée à une application de matrice collagénique cellularisée avec des cellules de la moelle. Cette matrice n'est pour l'instant pas modifiée avec des peptides d'adhésion. (cf. Etude MAGNUM). Nous avons montré récemment l'intérêt d'utiliser cette matrice collagénique cellularisée apposée sur le myocarde par rapport à la simple injection de cellules. (*cf. JC Chachques, JG Trainini, J. Mouras, O. Schussler. Myocardial assistance by grafting a new bioartificial upgraded myocardium (MAGNUM trial): Preliminary results AATS (2006); JC. Chachques, JC. Traini , O. Schussler. Myocardial Assistance by Grafting a new bioartificial upgraded myocardium (MAGNUM trial): Clinical feasibility AHA (2006))* Le développement de matrices de collagène modifiées comme nous le proposons devrait encore grandement améliorer les résultats dans un futur proche.

Cette invention se propose d'améliorer la thérapie cellulaire dans le domaine médecine et chirurgie thoracique et cardiovasculaire en utilisant des supports collagéniques modifiés avec des molécules d'adhésion avec, pour principale application, la réalisation d'un tissu cardiaque contractile. En plus des applications cardiovasculaires et thoraciques et de manière plus générale, ce support pourrait être utilisé pour la réalisation de tissu contractile spontanément ou sous stimulation électrique avec un intérêt pour la réalisation de tissu musculaire lisse ou squelettique afin de fabriquer par exemple des sphincters, un muscle vésical etc.).

Parmi les principales applications se trouvent l'utilisation de tels supports pour l'ingénierie tissulaire avec la fabrication de tissu de remplacement, reconstruction, patch, régénération tissulaire, accélération de la cicatrisation, prévention déhiscence, désunion, étanchéité air et/ou liquides, avec entre autre la réalisation de tissus : myocardique et/ou tissus valvulaire (en incluant les valves, l'appareil sous valvulaire et chambre de chasse), tissus trachéobronchique et pulmonaire, tissus vasculaire, tissus de reconstruction pariétal et péricardique. Inclus également l'utilisation de ce type de support pour la thérapie cellulaire avec possibilité que ce support soit injecté dans un tissus, dans une lumière vasculaire ou bronchique, dans un autre support ou dispositif médical ou chirurgical utilisé en chirurgie thoracique ou cardiovasculaire. Possibilité d'utiliser ce support pour réaliser un « pacemaker » biologique en associant des cellules ayant une activité rythmique intrinsèque comme des cardiomyocytes atriaux dissociés (cellules nodales y compris de sinus) d'origine embryonnaire, foetale ou adulte ou des cellules transformées ayant une activité rythmique implantées dans une matrice biodégradable pro-angiogénique et destinée à être implantées dans le coeur. L'association des peptides d'adhésion maintiendra la cellule au même endroit, favorisera sa survie, l'interaction entre les cellules avec une meilleure synchronisation entre elles et une meilleure intégration avec le tissu environnant.

Ceci pourrait conduire à une méthode pour l'ingénierie de tissus cardiaques en utilisant une population de cellules ayant ou pouvant développer des possibilités contractiles au cours de leur différenciation. Ensemencement des cellules dans l'échafaudage de matrice de cellules. Possibilité d' exposer la cellule à un ou des agents choisis pour favoriser la différenciation de la cellule où l'étape d'exposition peut être exécutée avant ou après l'étape d'implantation dans la matrice ou toutes les deux. La possibilité d'associer d'autres types cellulaires comme des cellules appartenant à la lignée endothéliale. Comme pour les cellules contractiles le support va permettre leur survie, leur différenciation. L'invention fournit un moyen de réaliser des constructions cardiaques en l'absence de gel tel que Matrigel™ (Becton-Dickinson) qui est extrait de tumeur et réduit la diffusion des nutriments obligeant à réaliser des tissus de moindre épaisseur. D'autre part, la différenciation terminale des cellules contractiles ne nécessite pas obligatoirement l'utilisation de l'électrostimulation chronique, de bioréacteurs, et des doses élevées du sérum xénogénique très immunogène. Il est également possible de réaliser des cultures de cellules sur des supports 2D contenant indifféremment de la fibrine/fibrinogène/collagène ou association et dont au moins l'un de ces constituants est modifié par la fixation de molécule d'adhésions. L'adhérence des cellules à ce support pourrait être contrôlée. Des cultures sur support de fibrines sont actuellement en développement ¹¹⁵. Différentes couches de cellules pourraient être superposées pour obtenir des tissus d'épaisseur plus importante ¹¹⁴. Des cellules endothéliales pourraient être associées afin de favoriser l'angiogénèse. Ces supports pourraient être appliqués sur le coeur en utilisant des systèmes comme des membranes de collagène pour faciliter cette application.

La construction peut être formée in vitro pendant plusieurs semaines et alors être directement implantée sur le myocarde en tant que pièce rapportée contractile ou à l'intérieur du myocarde visant à remplacer par exemple une partie ou la totalité du tissu myocardique. Alternativement la construction pourrait être implantée in vivo en position ectopique pour favoriser la différenciation des cellules et leur néovascularisation. De tels emplacements pourraient être par exemple les emplacements ectopiques bien vascularisés tels que la cavité péritonéale et pleurale, l'epiploon, muscle squelettique electrostimulé ou pas, cavité pleurale, péricarde, paroi. La géométrie interne du myocarde pourrait être fournie en enveloppant in situ le tissu autour de structures si possible non adhérentes et si non résorbable telles que prothèse en silicone ou toute autre polymère synthétique. Ces structures pourraient être à géométrie variable et pourraient prendre la forme d'une partie de la cavité ventriculaire. Ce dispositif pourrait être gonflable. Les variations de volume de ce dispositif pourraient être un moyen d'appliquer des stress mécaniques au tissus myocardique en voie de développement. D'autres stimuli tel qu'un stimulus électrique chronique pourraient également être appliqués. Plusieurs feuilles de tissu pourraient être superposées successivement pour construire un tissu plus épais lors de la première intervention ou lors d'intervention successives. La construction vascularisée peut être alors secondairement appliquée sur le coeur en greffon libre ou en greffon pédiculisé. mais il est aussi possible de remplacer la une partie ou l'épaisseur totale du myocarde. L'échafaudage cellularisé peut être fixé sur le coeur en employant par exemple des gels de collagène ou de fibrine. Ces approches peuvent également employer une thérapie cellulaire associée ou non à l'échafaudage 3D modifié ou pas par des peptides d'adhésion. Le patch ventriculaire peut-être une partie ou associé à un dispositif de contention du coeur qui vise à empêcher la dilatation cardiaque, favoriser un remodelage, améliorer la contractilité myocardique, régénération ou à traiter des conditions myocardiques. L'association du dispositif de contention myocardique à un support destiné à être cellularisé réalise une assistance cardiaque biologique. Il est possible également de concevoir un filet qui entoure le coeur et qui pourrait être cellularisé. L'utilisation de patch contractile comme prothèse aortique ou apposé sur l'aorte native synchronisé ou pas sur la fréquence cardiaque est une autre façon de réaliser une assistance cardiaque biologique.

Des matrices collagéniques pourraient être également utilisées pour la thérapie cellulaire. Les cellules peuvent être associés à des supports fonctionnalisés avec des molécules d'adhésion et réticulés in vitro ou in vivo. Dans d'autres cas, les cellules peuvent être injectées. Différentes formes de supports peuvent alors être utilisés comme des gels, hydrogels formés à partir de nanoparticules de collagènes, microsphères de collagène associées avec des nanoparticules organiques ou pas, collagène associé à des nanoparticules organiques ou pas etc.... L'invention se propose d'améliorer la biofonctionnalité d'échafaudages de fibrine seule ou associée avec du collagène par exemple en modifiant le fibrinogène, la fibrine et/ou le collagène par la fixation de molécules d'adhésion comme nous le proposons. L'association de motif RGD lors de la polymérisation de colle de fibrine a été proposée pour l'injection de cellule dans le coeur mais il s'agit de molécule d'adhésion non fixées et dont la fonctionnalité peut être améliorée (US Patent 20052761631). Les molécules d'adhésion peuvent être associées au fibrinogène ou au collagène in vitro. L'enzyme associée au moment de l'injection, généralement la thrombine en présence de calcium, induit la transformation du fibrinogène en fibrine et la formation du support 3D. Cet échafaudage de fibrine/fibrinogène/collagène éventuellement associé à d'autres composants pourrait être utilisée pour l'ingénierie tissulaire, la thérapie cellulaire ou pour réaliser différents dispositifs médicaux et/ou chirurgicaux pas seulement dans le domaine cardiovasculaire.

Parmi les dispositifs chirurgicaux/médicaux dans le domaine thoracique et cardiovasculaire qui seraient avantageusement améliorés on retrouve la réalisation de valve viable stentée ou pas construite à partir de support contenant du collagène ou fibrine/fibrinogène modifiés et cellularisés avec des cellules autologues ou pas (supports différents de ceux proposés comme les matrices synthétiques (US patents 20060253192, 20060246584), des anneaux valvulaires résorbables ou des anneaux viables avec possibilité de remodelage, filet de contention myocardique contenant du collagène ou associé à un support contenant du collagène ou assistance biologique externe visant à améliorer la contraction et/ou régénération et/ou remodelage et/ou contention myocardique et/ou à traiter des conditions myocardiques ou un myocarde pathologique et/ou à maintenir un support cellularisé, colles biologiques, membranes d'hémocompatibilité, réalisation de bioprothèse valvulaire cardiaques peu thrombogène, tube valvé, fabrication de prothèse, endoprothèse et stent vasculaire, de substitut vasculaire avec par exemple des support collagénique modifiés pour réaliser des greffons peu thrombogène (greffons vasculaire y compris greffons coronaires), réalisation de greffon vasculaire contractile spontanément ou sous stimulation électrique de telle greffons pourraient être utilisés comme assistance cardiaque, stent coronaires et endosinusien (drug eluted stent, stent résorbables, stent cellularisés) (association du RGD cyclique au composant métallique de drug eluted stent déjà proposé US patent 20050123582), modification du contenu collagénique endobronchique coil ou bypass avec également la possibilité d'y associer des cellules d'intérêts, prothèse de remplacement trachéobronchqiue, prothèse et stent endotracheal ou endobronchique, support pour la libération d'agent bioactif.

La biocompatibilité des supports collagéniques en général reconstitués à partir de fibres de collagène est considérablement améliorée si, après réticulation par la génipine, ils sont partiellement recouverts de protéoglycanes par les méthodes classiques utilisées déjà lors de la fixation de tissus par la génipine. Dans le cas de la fixation classique des supports collagéniques (tissus, support reconstitués ou dispositif contenant du collagène) par le glutararaldehyde la biocompatibilité est améliorée par une fixation ultérieure de protéoglycane par un réactif de couplage des protéines tel que la carbodiimide soluble, suivie éventuellement d'une réduction par le borohydrure de sodium ou le cyanoborohydrure de sodium. Le composant collagénique de ces supports peut être avantageusement fonctionnalisé avec des molécules d'adhésions.

Les supports collagéniques modifiés selon l'invention peuvent en outre trouver une application importante pour la thérapie cellulaire dans le muscle, l'engineering de tissu contractile spontanément ou sous stimulation (muscle lisse, squelettique, cardiaque), la thérapie cellulaire en général utilisant des cellules à potentiel contractile qui peuvent être éventuellement modifiées génétiquement pour libérer par exemple des agents bioactifs.

Possibilité d'utiliser les support de fibrine/fibrinogène avec ou sans collagène, modifiés ou pas pour : la thérapie cellulaire, l'ingénierie tissulaire et pour la réalisation de dispositifs médicaux/chirurgicaux en général.
1. Yasuda, T. et al. Quantitative analysis of survival of transplanted smooth muscle cells with real-time polymerase chain reaction. J Thorac Cardiovasc Surg 129, 904-11 (2005).
2. Whittaker, P., Muller-Ehmsen, J., Dow, J.S., Kedes, L.H. & Kloner, R.A. Development of abnormal tissue architecture in transplanted neonatal rat myocytes. Ann Thorac Surg 75, 1450-6 (2003).
3. Mangi, A.A. et al. Mesenchymal stem cells modified with Akt prevent remodeling and restore performance of infarcted hearts. Nat Med 9, 1195-201 (2003).
4. Leor, J., Amsalem, Y. & Cohen, S. Cells, scaffolds, and molecules for myocardial tissue engineering. Pharmacol Ther 105, 151-63 (2005).
5. Zimmermann, W.H., Melnychenko, I. & Eschenhagen, T. Engineered heart tissue for regeneration of diseased hearts. Biomaterials 25, 1639-47 (2004).
6. Eschenhagen, T. & Zimmermann, W.H. Engineering myocardial tissue. Circ Res 97, 1220-31 (2005).
7. Nugent, H.M. & Edelman, E.R. Tissue engineering therapy for cardiovascular disease. Circ Res 92, 1068-78 (2003).
8. Shachar, M. & Cohen, S. Cardiac tissue engineering, ex-vivo: design principles in biomaterials and bioreactors. Heart Fail Rev 8, 271-6 (2003).
9. Suuronen, E.J. et al. Tissue-engineered injectable collagen-based matrices for improved cell delivery and vascularization of ischemic tissue using CD133+ progenitors expanded from the peripheral blood. Circulation 114, 1138-44 (2006).
10. Qian, H., Yang, Y., Huang, J., Dou, K. & Yang, G. Cellular cardiomyoplasty by catheter-based infusion of stem cells in clinical settings. Transpl Immunol 16, 135-47 (2006).
11. Kumaran, V., Joseph, B., Benten, D. & Gupta, S. Integrin and extracellular matrix interactions regulate engraftment of transplanted hepatocytes in the rat liver. Gastroenterology 129, 1643-53 (2005).
12. MacDonald, R.A., Laurenzi, B.F., Viswanathan, G., Ajayan, P.M. & Stegemann, J.P. Collagen-carbon nanotube composite materials as scaffolds in tissue engineering. J Biomed Mater Res A 74, 489-96 (2005).
13. Griffith, L.G. & Swartz, M.A. Capturing complex 3D tissue physiology in vitro. Nat Rev Mol Cell Biol 7, 211-24 (2006).
14. Vogel, V. & Sheetz, M. Local force and geometry sensing regulate cell functions. Nat Rev Mol Cell Biol 7, 265-75 (2006).
15. Badylak, S.F. Xenogeneic extracellular matrix as a scaffold for tissue reconstruction. Transpl Immunol 12, 367-77 (2004).
16. Samarel, A.M. Costameres, focal adhesions, and cardiomyocyte mechanotransduction. Am J Physiol Heart Circ Physiol 289, H2291-301 (2005).
17. Kuppuswamy, D. Importance of integrin signaling in myocyte growth and survival. Circ Res 90, 1240-2 (2002).
18. Meredith, J. & Schwartz, M. Integrins, adhesion and apoptosis, 146-150 (1997).
19. Walker, J.L., Fournier, A.K. & Assoian, R.K. Regulation of growth factor signaling and cell cycle progression by cell adhesion and adhesion-dependent changes in cellular tension. Cytokine Growth Factor Rev 16, 395-405 (2005).
20. Chen, S.S., Fitzgerald, W., Zimmerberg, J., Kleinman, H.K. & Margolis, L. Cell-cell and cell-extracellular matrix interactions regulate embryonic stem cell differentiation. Stem Cells 25, 553-61 (2007).
21. Liu, W.F. & Chen, S.C. Engineering biomaterials to control cell function. Materials Today 8, 28-35 (2005).
22. Hersel, U., Dahmen, C. & Kessler, H. RGD modified polymers: biomaterials for stimulated cell adhesion and beyond. Biomaterials 24, 4385-415 (2003).
23. Beer, J.H., Springer, K.T. & Coller, B.S. Immobilized Arg-Gly-Asp (RGD) peptides of varying lengths as structural probes of the platelet glycoprotein IIb/IIIa receptor. Blood 79, 117-28 (1992).
24. Craig, W.S., Cheng, S., Mullen, D.G., Blevitt, J. & Pierschbacher, M.D. Concept and progress in the development of RGD-containing peptide pharmaceuticals. Biopolymers 37, 157-75 (1995).
25. Katsumi, A., Orr, A.W., Tzima, E. & Schwartz, M.A. Integrins in mechanotransduction. J Biol Chem 279, 12001-4 (2004).
26. Reinhart-King, C.A., Dembo, M. & Hammer, D.A. The dynamics and mechanics of endothelial cell spreading. Biophys J 89, 676-89 (2005).
27. Simon, A. & Durrieu, M.C. Strategies and results of atomic force microscopy in the study of cellular adhesion. Micron 37, 1-13 (2006).
28. Engler, A.J. et al. Myotubes differentiate optimally on substrates with tissue-like stiffness: pathological implications for soft or stiff microenvironments. J Cell Biol 166, 877-87 (2004).
29. Deroanne, C.F., Lapiere, C.M. & Nusgens, B.V. In vitro tubulogenesis of endothelial cells by relaxation of the coupling extracellular matrix-cytoskeleton. Cardiovasc Res 49, 647-58 (2001).
30. Leor, J. & Cohen, S. Myocardial tissue engineering: creating a muscle patch for a wounded heart. Ann N Y Acad Sci 1015, 312-9 (2004).
31. Davis, M.E. et al. Injectable self-assembling peptide nanofibers create intramyocardial microenvironments for endothelial cells. Circulation 111, 442-50 (2005).
32. Narmoneva, D.A., Vukmirovic, R., Davis, M.E., Kamm, R.D. & Lee, R.T. Endothelial cells promote cardiac myocyte survival and spatial reorganization: implications for cardiac regeneration. Circulation 110, 962-8 (2004).
33. Weadock, K.S., Miller, E.J., Keuffel, E.L. & Dunn, M.G. Effect of physical crosslinking methods on collagen-fiber durability in proteolytic solutions. J Biomed Mater Res 32, 221-6 (1996).
34. Cornwell, K.G., Lei, P., Andreadis, S.T. & Pins, G.D. Crosslinking of discrete self-assembled collagen threads: Effects on mechanical strength and cell-matrix interactions. J Biomed Mater Res A 80, 362-71 (2007).
35. Kannan, R.Y., Salacinski, H.J., Sales, K., Butler, P. & Seifalian, A.M. The roles of tissue engineering and vascularisation in the development of micro-vascular networks: a review. Biomaterials 26, 1857-75 (2005).
36. Ilan, N., Mahooti, S. & Madri, J.A. Distinct signal transduction pathways are utilized during the tube formation and survival phases of in vitro angiogenesis. J Cell Sci 111 (Pt 24), 3621-31 (1998).
37. Satake, S., Kuzuya, M., Ramos, M.A., Kanda, S. & Iguchi, A. Angiogenic stimuli are essential for survival of vascular endothelial cells in three-dimensional collagen lattice. Biochem Biophys Res Commun 244, 642-6 (1998).
38. Ranta, V., Mikkola, T., Ylikorkala, O., Viinikka, L. & Orpana, A. Reduced viability of human vascular endothelial cells cultured on Matrigel. J Cell Physiol 176, 92-8 (1998).
39. Goto, F., Goto, K., Weindel, K. & Folkman, J. Synergistic effects of vascular endothelial growth factor and basic fibroblast growth factor on the proliferation and cord formation of bovine capillary endothelial cells within collagen gels [see comments]. Laboratory Investigation 69, 508-17 (1993).
40. Radisic, M. et al. Functional assembly of engineered myocardium by electrical stimulation of cardiac myocytes cultured on scaffolds. Proc Natl Acad Sci U S A 101, 18129-34 (2004).
41. Radisic, M. et al. Medium perfusion enables engineering of compact and contractile cardiac tissue. Am J Physiol Heart Circ Physiol 286, H507-16 (2004).
42. Souren, J.E., Peters, R.C. & Van Wijk, R. Collagen gels populated with rat neonatal heart cells can be used for optical recording of rhythmic contractions which also show ECG-like potentials. Experientia 50, 712-6 (1994).
43. Souren, J.E., Schneijdenberg, C., Verkleij, A.J. & Van Wijk, R. Factors controlling the rhythmic contraction of collagen gels by neonatal heart cells. In Vitro Cell Dev Biol 28A, 199-204 (1992).
44. Zimmermann, W.H. et al. Three-dimensional engineered heart tissue from neonatal rat cardiac myocytes. Biotechnol Bioeng 68, 106-14 (2000).
45. Zimmermann, W.H. et al. Engineered heart tissue grafts improve systolic and diastolic function in infarcted rat hearts. Nat Med 12, 452-8 (2006).
46. Eschenhagen, T., Didie, M., Heubach, J., Ravens, U. & Zimmermann, W.H. Cardiac tissue engineering. Transpl Immunol 9, 315-21 (2002).
47. Zimmermann, W.H. et al. Cardiac grafting of engineered heart tissue in syngenic rats. Circulation 106, I151-7 (2002).
48. von Wnuck Lipinski, K. et al. Integrin-mediated transcriptional activation of inhibitor of apoptosis proteins protects smooth muscle cells against apoptosis induced by degraded collagen. Circ Res 98, 1490-7 (2006).
49. Meinel, L. et al. Engineering cartilage-like tissue using human mesenchymal stem cells and silk protein scaffolds. Biotechnol Bioeng 88, 379-91 (2004).
50. Coyle, C.H., Mendralla, S., Lanasa, S. & Kader, K.N. Endothelial Cell Seeding onto Various Biomaterials Causes Superoxide-induced Cell Death. J Biomater Appl (2006).
51. Grzesiak, J.J., Pierschbacher, M.D., Amodeo, M.F., Malaney, T.I. & Glass, J.R. Enhancement of cell interactions with collagen/glycosaminoglycan matrices by RGD derivatization. Biomaterials 18, 1625-32 (1997).
52. Dagalakis, N., Flink, J., Stasikelis, P., Burke, J.F. & Yannas, I.V. Design of an artificial skin. Part III. Control of pore structure. J Biomed Mater Res 14, 511-28 (1980).
53. Yannas, I.V., Lee, E., Orgill, D.P., Skrabut, E.M. & Murphy, G.F. Synthesis and characterization of a model extracellular matrix that induces partial regeneration of adult mammalian skin. Proc Natl Acad Sci U S A 86, 933-7 (1989).
54. Suuronen, E.J. et al. Innervated human corneal equivalents as in vitro models for nerve-target cell interactions. Faseb J 18, 170-2 (2004).
55. Kofidis, T. et al. Clinically established hemostatic scaffold (tissue fleece) as biomatrix in tissue- and organ-engineering research. Tissue Eng 9, 517-23 (2003).
56. Park, H., Radisic, M., Lim, J.O., Chang, B.H. & Vunjak-Novakovic, G. A novel composite scaffold for cardiac tissue engineering. In Vitro Cell Dev Biol Anim 41, 188-96 (2005).
57. Chen, R.N., Ho, H.O. & Sheu, M.T. Characterization of collagen matrices crosslinked using microbial transglutaminase. Biomaterials 26, 4229-35 (2005).
58. Goissis, G. et al. Biocompatibility studies of anionic collagen membranes with different degree of glutaraldehyde cross-linking. Biomaterials 20, 27-34 (1999).
59. Park, S.N., Park, J.C., Kim, H.O., Song, M.J. & Suh, H. Characterization of porous collagen/hyaluronic acid scaffold modified by 1-ethyl-3-(3-dimethylaminopropyl)carbodiimide cross-linking. Biomaterials 23, 1205-12 (2002).
60. Zandonella, C. Tissue engineering: The beat goes on. Nature 421, 884-6 (2003).
61. Jockenhoevel, S. et al. Fibrin gel -- advantages of a new scaffold in cardiovascular tissue engineering. Eur J Cardiothorac Surg 19, 424-30 (2001).
62. Christman, K.L., Fok, H.H., Sievers, R.E., Fang, Q. & Lee, R.J. Fibrin glue alone and skeletal myoblasts in a fibrin scaffold preserve cardiac function after myocardial infarction. Tissue Eng 10, 403-9 (2004).
63. Christman, K.L. et al. Injectable fibrin scaffold improves cell transplant survival, reduces infarct expansion, and induces neovasculature formation in ischemic myocardium. J Am Coll Cardiol 44, 654-60 (2004).
64. Mol, A. et al. Fibrin as a cell carrier in cardiovascular tissue engineering applications. Biomaterials 26, 3113-21 (2005).
65. Rowe, S.L., Lee, S. & Stegemann, J.P. Influence of thrombin concentration on the mechanical and morphological properties of cell-seeded fibrin hydrogels. Acta Biomater 3, 59-67 (2007).
66. Liu, W. et al. Fibrin fibers have extraordinary extensibility and elasticity. Science 313, 634 (2006).
67. Boublik, J. et al. Mechanical properties and remodeling of hybrid cardiac constructs made from heart cells, fibrin, and biodegradable, elastomeric knitted fabric. Tissue Eng 11, 1122-32 (2005).
68. Beier, J.P. et al. Tissue engineering of injectable muscle: three-dimensional myoblast-fibrin injection in the syngeneic rat animal model. Plast Reconstr Surg 118, 1113-21; discussion 1122-4 (2006).
69. Williams, C., Johnson, S.L., Robinson, P.S. & Tranquillo, R.T. Cell sourcing and culture conditions for fibrin-based valve constructs. Tissue Eng 12, 1489-502 (2006).
70. McKenna, C.J. et al. Fibrin-film stenting in a porcine coronary injury model: efficacy and safety compared with uncoated stents. J Am Coll Cardiol 31, 1434-8 (1998).
71. Gutierrez San Roman, C. et al. Long-term assessment of the treatment of recurrent tracheoesophageal fistula with fibrin glue associated with diathermy. J Pediatr Surg 41, 1870-3 (2006).
72. Schense, J.C., Bloch, J., Aebischer, P. & Hubbell, J.A. Enzymatic incorporation of bioactive peptides into fibrin matrices enhances neurite extension. Nat Biotechnol 18, 415-9 (2000).
73. Hall, H., Baechi, T. & Hubbell, J.A. Molecular properties of fibrin-based matrices for promotion of angiogenesis in vitro. Microvasc Res 62, 315-26 (2001).
74. Korff, T. & Augustin, H.G. Tensional forces in fibrillar extracellular matrices control directional capillary sprouting. J Cell Sci 112 (Pt 19), 3249-58 (1999).
75. Eyrich, D. et al. Long-term stable fibrin gels for cartilage engineering. Biomaterials 28, 55-65 (2007).
76. Cummings, C.L., Gawlitta, D., Nerem, R.M. & Stegemann, J.P. Properties of engineered vascular constructs made from collagen, fibrin, and collagen-fibrin mixtures. Biomaterials 25, 3699-706 (2004).
77. Rowe, S.L. & Stegemann, J.P. Interpenetrating collagen-fibrin composite matrices with varying protein contents and ratios. Biomacromolecules 7, 2942-8 (2006).
78. Sarin, V., Gaffin, R.D., Meininger, G.A. & Muthuchamy, M. Arginine-glycine-aspartic acid (RGD)-containing peptides inhibit the force production of mouse papillary muscle bundles via alpha 5 beta 1 integrin. J Physiol 564, 603-17 (2005).
79. Balasubramanian, S. & Kuppuswamy, D. RGD-containing peptides activate S6K1 through beta3 integrin in adult cardiac muscle cells. J Biol Chem 278, 42214-24 (2003).
80. Boateng, S.Y. et al. RGD and YIGSR synthetic peptides facilitate cellular adhesion identical to that of laminin and fibronectin but alter the physiology of neonatal cardiac myocytes. Am J Physiol Cell Physiol 288, C30-8 (2005).
81. Blindt, R. et al. A novel drug-eluting stent coated with an integrin-binding cyclic Arg-Gly-Asp peptide inhibits neointimal hyperplasia by recruiting endothelial progenitor cells. J Am Coll Cardiol 47, 1786-95 (2006).
82. Myles, J.L., Burgess, B.T. & Dickinson, R.B. Modification of the adhesive properties of collagen by covalent grafting with RGD peptides. J Biomater Sci Polym Ed 11, 69-86 (2000).
83. Lindberg, K. & Badylak, S.F. Porcine small intestinal submucosa (SIS): a bioscaffold supporting in vitro primary human epidermal cell differentiation and synthesis of basement membrane proteins. Bums 27, 254-66 (2001).
84. Badylak, S.F., Record, R., Lindberg, K., Hodde, J. & Park, K. Small intestinal submucosa: a substrate for in vitro cell growth. J Biomater Sci Polym Ed 9, 863-78 (1998).
85. Pierschbacher, M.D. & Ruoslahti, E. Cell attachment activity of fibronectin can be duplicated by small synthetic fragments of the molecule. Nature 309, 30-3 (1984).
86. Pfaff, M., McLane, M.A., Beviglia, L., Niewiarowski, S. & Timpl, R. Comparison of disintegrins with limited variation in the RGD loop in their binding to purified integrins alpha Ilb beta 3, alpha V beta 3 and alpha 5 beta 1 and in cell adhesion inhibition. Cell Adhes Commun 2, 491-501 (1994).
87. Iwamoto, Y. et al. YIGSR, a synthetic laminin pentapeptide, inhibits experimental metastasis formation. Science 238, 1132-4 (1987).
88. Tashiro, K. et al. A synthetic peptide containing the IKVAV sequence from the A chain of laminin mediates cell attachment, migration, and neurite outgrowth. J Biol Chem 264, 16174-82 (1989).
89. Liesi, P., Narvanen, A., Soos, J., Sariola, H. & Snounou, G. Identification of a neurite outgrowth-promoting domain of laminin using synthetic peptides. FEBS Lett 244, 141-8 (1989).
90. Massia, S.P. & Hubbell, J.A. Vascular endothelial cell adhesion and spreading promoted by the peptide REDV of the IIICS region of plasma fibronectin is mediated by integrin alpha 4 beta 1. J Biol Chem 267, 14019-26 (1992).
91. Altroff, H. et al. The eighth FIII domain of human fibronectin promotes integrin alpha5beta1 binding via stabilization of the ninth FIII domain. J Biol Chem 276, 38885-92 (2001).
92. Wong, J.Y., Weng, Z., Moll, S., Kim, S. & Brown, C.T. Identification and validation of a novel cell-recognition site (KNEED) on the 8th type III domain of fibronectin. Biomaterials 23, 3865-70 (2002).
93. Dee, K.C., Andersen, T.T. & Bizios, R. Design and function of novel osteoblast-adhesive peptides for chemical modification of biomaterials. J Biomed Mater Res 40, 371-7 (1998).
94. Rezania, A. & Healy, K.E. Biomimetic peptide surfaces that regulate adhesion, spreading, cytoskeletal organization, and mineralization of the matrix deposited by osteoblast-like cells. Biotechnol Prog 15, 19-32 (1999).
95. Rezania, A. & Healy, K.E. Integrin subunits responsible for adhesion of human osteoblast-like cells to biomimetic peptide surfaces. J Orthop Res 17, 615-23 (1999).
96. Mann, B.K. & West, J.L. Cell adhesion peptides alter smooth muscle cell adhesion, proliferation, migration, and matrix protein synthesis on modified surfaces and in polymer scaffolds. J Biomed Mater Res 60, 86-93 (2002).
97. Emsley, J., Knight, C.G., Farndale, R.W., Barnes, M.J. & Liddington, R.C., Structural basis of collagen recognition by integrin alpha2beta1. Cell 101, 47-56 (2000).
98. Lee, W.K. et al. Improved calcification resistance and biocompatibility of tissue patch grafted with sulfonated PEO or heparin after glutaraldehyde fixation. J Biomed Mater Res 58, 27-35 (2001).
99. Chang, Y., Liang, H.C., Wei, H.J., Chu, C.P. & Sung, H.W. Tissue regeneration patterns in acellular bovine pericardia implanted in a canine model as a vascular patch. J Biomed Mater Res A 69, 323-33 (2004).
100. Chang, Y., Tsai, C.C., Liang, H.C. & Sung, H.W. In vivo evaluation of cellular and acellular bovine pericardia fixed with a naturally occurring crosslinking agent (genipin). Biomaterials 23, 2447-57 (2002).
101. Levenberg, S. et al. Engineering vascularized skeletal muscle tissue. Nat Biotechnol 23, 879-84 (2005).
102. Radisic, M. et al. High-density seeding of myocyte cells for cardiac tissue engineering. Biotechnol Bioeng 82, 403-14 (2003).
103. Coirault, C., Chemla, D., Suard, I., Pourny, J.C. & Lecarpentier, Y. Sarcomere relaxation in hamster diaphragm muscle. J Appl Physiol 81, 858-65 (1996).
104. Lecarpentier, Y. et al. Mechanics, energetics, and crossbridge kinetics of rabbit diaphragm during congestive heart failure. Faseb J 12, 981-9 (1998).
105. Lecarpentier, Y. et al. Real-time kinetics of sarcomere relaxation by laser diffraction. Circ Res 56, 331-9 (1985).
106. Black, A.F., Berthod, F., L'heureux, N., Germain, L. & Auger, F.A. In vitro reconstruction of a human capillary-like network in a tissue-engineered skin equivalent. Faseb Journal 12, 1331-40 (1998).
107. Baatout, S. Endothelial differentiation using Matrigel (review). Anticancer Research 17, 451-5 (1997).
108. Montesano, R., Pepper, M.S. & Orci, L. Paracrine induction of angiogenesis in vitro by Swiss 3T3 fibroblasts. Journal of Cell Science 105 (Pt 4), 1013-24 (1993).
109. De Coppi, P. et al. Angiogenic gene-modified muscle cells for enhancement of tissue formation. Tissue Eng 11, 1034-44 (2005).
110. Arbiser, J.L. et al. Oncogenic H-ras stimulates tumor angiogenesis by two distinct pathways. proceedings of the national academy of sciences of the united states of america 94, 861-6 (1997).
111. Cherqui, S. et al. Isolation and angiogenesis by endothelial progenitors in the fetal liver. Stem Cells 24, 44-54 (2006).
112. Hildbrand, P. et al. The role of angiopoietins in the development of endothelial cells from cord blood CD34+ progenitors. Blood 104, 2010-9 (2004).
113. Crisa, L. et al. Human cord blood progenitors sustain thymic T-cell development and a novel form of angiogenesis. Blood 94, 3928-40 (1999).
114. Shimizu, T. et al. Polysurgery of cell sheet grafts overcômes diffusion limits to produce thick, vascularized myocardial tissues. Faseb J 20, 708-10 (2006).
115. Furuta, A. et al. Pulsatile cardiac tissue grafts using a novel three-dimensional cell sheet manipulation technique functionally integrates with the host heart, in vivo. Circ Res 98, 705-12 (2006).

## Revendications

1. Support tridimensionnel solide pour la colonisation, la survie, la différentiation terminale de cellules à potentiel contractile, et ayant une activité contractile spontanée ou obtenue par stimulation, **caractérisé en ce que** ledit support tridimensionnel est constitué d'une éponge de collagène dans laquelle :
a. le collagène est réticulé,
b. le collagène est en partie ou intégralement modifié par la fixation covalente de molécules d'adhésion ; et
c. le collagène est exempt de tout extrait tumoral;
ledit support étant destiné à la thérapie cellulaire ou à l'ingénierie tissulaire dans le domaine médecine/chirurgie thoracique et cardiovasculaire.

2. Support collagénique tridimensionnel selon la revendication 1, **caractérisé en ce que** les molécules d'adhésion comprennent le motif RGD.

3. Support collagénique tridimensionnel selon l'une quelconque des revendications 1 ou 2, **caractérisé en ce que** le collagène est purifié à partir d'un tissu collagénique.

4. Support collagénique tridimensionnel selon l'une quelconque des revendications 1 à 3, **caractérisé en ce qu'**il contient ou qu'il est associé à une ou plusieurs molécules ou agents bioactifs.

5. Support collagénique tridimensionnel selon l'une quelconque des revendications 1 à 4, **caractérisé en ce qu'**il contient au moins un contingent cellulaire.

6. Support collagénique tridimensionnel selon la revendication 5, **caractérisé en ce que** les cellules dudit au moins un contingent cellulaire sont des cellules à potentiel contractile, rythmique ou angiogénique.

7. Support collagénique tridimensionnel selon l'une quelconque des revendications précédentes, **caractérisé en ce que** les molécules d'adhésion et/ou les molécules ou agents bioactifs sont liés par l'intermédiaire de radicaux espaceurs et la nature hydrophile, la flexibilité et la longueur des radicaux espaceurs liant les molécules d'adhésion et/ou les molécules ou agents bioactifs sont choisis pour optimiser les disponibilités spatiales et faciliter la présentation et l'interaction avec des cellules d'intérêt.

8. Support collagénique tridimensionnel selon l'une quelconque des revendications précédentes, **caractérisé en ce que** la fixation covalente des molécules d'adhésion met en jeu un réactif de couplage hétérobifonctionnel, pour relier de façon covalente et unidirectionnelle des groupements amines primaires des molécules d'adhésion à des groupements amines primaires présents dans le support tridimensionnel.

9. Support collagénique tridimensionnel selon l'une quelconque des revendications précédentes, **caractérisé en ce que** le collagène est réticulé par un traitement physique choisi parmi l'irradiation UV, l'irradiation par faisceaux ionisants, ou la déshydration et traitement par la chaleur (dehydrothermal crosslinking, DHT).

10. Support collagénique tridimensionnel selon l'une quelconque des revendications précédentes, **caractérisé en ce qu'**il est réticulé par différents procédés choisis parmi la réticulation par le glutaraldéhyde, un traitement par des protéoglycanes et un traitement par un réactif réducteur des bases de Schiff, ou leur association.

11. Support collagénique tridimensionnel selon l'une quelconque des revendications 1 à 9, associé ou non à des protéoglycanes, **caractérisé en ce qu'**il est réticulé par l'action de la génipine.

12. Procédé de fabrication dans le domaine médecine/chirurgie d'un support collagénique tridimensionnel destiné à la thérapie cellulaire et/ou à l'ingénierie de tissu contractile, **caractérisé en ce qu'**il comprend les étapes suivantes :
a) une partie ou l'intégralité du contenu du support est formée de collagène éventuellement réticulé;
b) le contenu du support a été en partie ou intégralement modifié par la fixation covalente de molécules d'adhésion ;
c) le support a été aussi réticulé par le glutaraldéhyde et un réactif réducteur des bases de Schiff ou par la génipine.

13. Procédé selon la revendication 12, **caractérisé en ce qu'**il comprend une étape supplémentaire d) dans laquelle le support a été traité ou non par des protéoglycanes.

14. Procédé selon l'une quelconque des revendications 12 ou 13, faisant appel à un agent de couplage hétérobifonctionnel tel que sulfo-LC-SPDP pour relier de façon covalente et unidirectionnelle des groupements amines primaires des molécules d'adhésion et/ou des molécules bioactives à des groupements amines primaires présents dans le support tridimensionnel.

15. Utilisation d'un support collagénique tridimensionnel selon l'une quelconque des revendications 1 à 11, ou obtenu par le procédé selon l'une quelconque des revendications 12 à 14, dans la réalisation de différents dispositifs du domaine médecine/chirurgie cardio-thoracique et vasculaire tels qu'un patch tissulaire, un muscle papillaire pour traiter des nécroses de pilier après infarctus, une prothèse vasculaire, un dispositif visant à traiter des conditions myocardiques qui associe a) un système permettant de contrôler le remodelage ou de limiter la distension ventriculaire par contention, b) un système améliorant la contractilité myocardique, un dispositif contractile pour la libération de principe actif, une colle biologique, un support pour la thérapie cellulaire et l'ingénierie de tissu dans le domaine thoracique et cardiovasculaire.

## Patentansprüche

1. Dreidimensionaler fester Träger für die Kolonisierung, das Überleben, die endgültige Differenzierung von Zellen mit kontraktilem Potenzial und mit einer kontraktilen Aktivität, die spontan ist oder durch Stimulation erhalten wird, **dadurch gekennzeichnet, dass** der dreidimensionale Träger aus einem Kollagenschwamm besteht, wobei:
a. das Kollagen vernetzt ist,
b. das Kollagen zum Teil oder vollständig durch die kovalente Bindung von Adhäsionsmolekülen modifiziert ist und
c. das Kollagen frei von jeglichem Tumorextrakt ist,
wobei der Träger für die Zelltherapie oder das Gewebeengineering auf dem Gebiet der Thorax- und Kardiovaskulärmedizin/-chirurgie bestimmt ist.

2. Dreidimensionaler Kollagenträger nach Anspruch 1, **dadurch gekennzeichnet, dass** die Adhäsionsmoleküle das RGD-Motiv umfassen.

3. Dreidimensionaler Kollagenträger nach einem der Ansprüche 1 oder 2, **dadurch gekennzeichnet, dass** das Kollagen aus einem kollagenhaltigen Gewebe gereinigt ist.

4. Dreidimensionaler Kollagenträger nach einem der Ansprüche 1 bis 3, **dadurch gekennzeichnet, dass** er ein oder mehrere bioaktive Moleküle oder Mittel umfasst oder damit assoziiert ist.

5. Dreidimensionaler Kollagenträger nach einem der Ansprüche 1 bis 4, **dadurch gekennzeichnet, dass** er mindestens einen Zellanteil umfasst.

6. Dreidimensionaler Kollagenträger nach Anspruch 5, **dadurch gekennzeichnet, dass** die Zellen des mindestens einen Zellanteils Zellen mit kontraktilem, rhythmischem oder angiogenem Potenzial sind.

7. Dreidimensionaler Kollagenträger nach einem der vorhergehenden Ansprüche, **dadurch gekennzeichnet, dass** die Adhäsionsmoleküle und/oder die bioaktiven Moleküle oder Mittel mithilfe von Spacerresten gebunden sind und dass die hydrophile Natur, die Flexibilität und die Länge der Spacerreste, die die Adhäsionsmoleküle und/oder die bioaktiven Moleküle oder Mittel binden, so ausgewählt sind, dass die räumlichen Verfügbarkeiten optimiert werden und die Darbietung und die Wechselwirkung mit Zellen von Interesse erleichtert ist.

8. Dreidimensionaler Kollagenträger nach einem der vorhergehenden Ansprüche, **dadurch gekennzeichnet, dass** die kovalente Bindung der Adhäsionsmoleküle ein heterobifunktionelles Kupplungsreagenz zum kovalenten und unidirektionalen Binden primärer Amingruppen der Adhäsionsmoleküle an in dem dreidimensionalen Träger vorhandene primäre Amingruppen beinhaltet.

9. Dreidimensionaler Kollagenträger nach einem der vorhergehenden Ansprüche, **dadurch gekennzeichnet, dass** das Kollagen durch eine physikalische Behandlung, ausgewählt aus UV-Bestrahlung, Bestrahlung mit ionisierenden Strahlen oder Dehydratisierung und Behandlung mit Wärme (dehydrothermische Vernetzung, DHT), vernetzt wird.

10. Dreidimensionaler Kollagenträger nach einem der vorhergehenden Ansprüche, **dadurch gekennzeichnet, dass** es durch verschiedene Verfahren, ausgewählt aus der Vernetzung durch Glutaraldehyd, einer Behandlung mit Proteoglykanen und einer Behandlung mit einem Schiff-Basen-Reduktionsmittel oder deren Kombination, vernetzt wird.

11. Dreidimensionaler Kollagenträger nach einem der Ansprüche 1 bis 9, der mit Proteoglykanen assoziiert ist oder nicht, **dadurch gekennzeichnet, dass** er durch die Einwirkung von Genipin vernetzt wird.

12. Verfahren zur Herstellung eines dreidimensionalen Kollagenträgers auf dem Gebiet der Medizin/Chirurgie, der für die Zelltherapie oder das Engineering von kontraktilem Gewebe bestimmt ist, **dadurch gekennzeichnet, dass** es die folgenden Schritte umfasst:
a) ein Teil oder die Gesamtheit des Inhalts des Trägers wird aus gegebenenfalls vernetztem Kollagen hergestellt,
b) der Inhalt des Trägers wurde zum Teil oder vollständig durch die kovalente Bindung von Adhäsionsmolekülen modifiziert,
c) der Träger wurde auch durch Glutaraldehyd und ein Schiff-Basen-Reduktionsmittel oder durch Genipin vernetzt.

13. Verfahren nach Anspruch 12, **dadurch gekennzeichnet, dass** es einen zusätzlichen Schritt (d) umfasst, in dem der Träger mit Proteoglykanen behandelt wurde oder nicht.

14. Verfahren nach einem der Ansprüche 12 oder 13, das ein heterobifunktionelles Kupplungsmittel, wie Sulfo-LC-SPDP, zum kovalenten und unidirektionalen Binden von primären Amingruppen der Adhäsionsmoleküle und/oder der bioaktiven Moleküle an in dem dreidimensionalen Träger vorhandene primäre Amingruppen verwendet.

15. Verwendung eines dreidimensionalen Kollagenträgers nach einem der Ansprüche 1 bis 11 oder erhalten durch das Verfahren nach einem der Ansprüche 12 bis 14 bei der Herstellung verschiedener Vorrichtungen auf dem Gebiet der Herz-Thorax- und Gefäßmedizin/-chirurgie, wie ein Gewebepflaster, ein Papillarmuskel zum Behandeln von Papillarmuskelnekrosen nach einem Infarkt, eine Gefäßprothese, eine Vorrichtung zur Behandlung von Myokardzuständen, die Folgendes kombiniert: a) ein System, das die Kontrolle der Remodellierung oder die Einschränkung der Ventrikelüberdehnung durch Eindämmung gestattet, b) ein System, das die Myokardkontraktilität verbessert, eine kontraktile Vorrichtung zur Freisetzung von Wirkstoff, ein biologischer Klebstoff, ein Träger für die Zelltherapie und das Gewebeengineering auf dem thorakalen und dem kardiovaskulären Gebiet.

## Claims

1. A three-dimensional solid scaffold for colonization, survival, terminal differentiation of cells with contractile potential, and having either a spontaneous contractile activity or obtained by stimulation, **characterized in that** said three-dimensional scaffold is made of a collagen sponge in which:
a) the collagen is cross-linked,
b) the collagen is partly or integrally modified by covalent fixation of a covalent adhesion molecule; and
c) the collagen is free of any tumoral extract; said scaffold being intended for cell therapy or tissue engineering in the thoracic and cardiovascular medical/surgical field.

2. A three-dimensional collagenic scaffold according to claim 1, **characterized in that** the adhesion molecules comprise a RGD unit.

3. A three-dimensional collagenic scaffold according to any one of claims 1 or 2, **characterized in that** the collagen is purified from a collagenic tissue.

4. A three-dimensional collagenic scaffold according to any one of claims 1 to 3, **characterized in that** it contains or is associated with one or more bioactive molecules or agents.

5. A three-dimensional collagenic scaffold according to any one of claims 1 to 4, **characterized in that** it contains at least one cellular contingent.

6. A three-dimensional collagenic scaffold according to claim 5, **characterized in that** the cells of said at least one cellular contingent are cells with contractile, rhythmic or angiogenic potential.

7. A three-dimensional collagenic scaffold according to any one of the preceding claims, **characterized in that** the adhesion molecules and/or the bioactive molecules or agents are linked through spacer radicals and the hydrophilic nature, the flexibility and the length of the spacer radicals linking the adhesion molecules and/or the bioactive molecules or agents are selected to optimize the spatial availabilities and facilitate the presentation and interaction with cells of interest.

8. A three-dimensional collagenic scaffold according to any one of the preceding claims, **characterized in that** the covalent binding of the adhesion molecules uses a heterobifunctional coupling reagent, to covalently and unidirectionnally link primary amine groups of the adhesion molecules to primary amine groups present in the three-dimensional scaffold..

9. A three-dimensional collagenic scaffold according to any of the preceding claims, **characterized in that** the collagen is cross-linked by means of a physical treatment selected form UV irradiation, irradiation with ionizing beams, or dehydration and heat treatment (dehydrothermal crosslinking, DHT).

10. A three-dimensional collagenic scaffold according to any of the preceding claims, **characterized in that** it is cross-linked through different processes selected from cross-linking with glutaraldehyde, a treatment with proteoglycans, and a treatment with a Schiff base-reducing reactant, or their association.

11. A three-dimensional collagenic scaffold according to any of claims 1 to 9, whether or not associated with proteoglycans, **characterized in that** it is cross-linked with genipin.

12. A method for making within the medical/chirurgical field a three-dimensional collagenic scaffold intended for cellular therapy and/or engineery of contractile tissue **characterized in that** it comprises the following steps: a) part of or the whole content of the scaffold is made of optionally cross-linked collagen; b) the scaffold content has been partially or fully modified by the covalent binding of adhesion molecules; c) the scaffold has also been cross-linked with glutaraldehyde and a Schiff base-reducing reactant or with genipin.

13. A process according to claim 12, **characterized in that** it comprises an additional step b) in which the scaffold has been or not treated by proteoglycans.

14. A process according to any of claims 12 or 13, using a heterobifunctional coupling agent such as suffo-LC-SPDP to link covalently and unidirectionally primary amine groups of adhesion molecules and/or bioactive molecules to primary amine groups present in the three-dimensional scaffold.

15. Use of a three-dimensional scaffold according to any one of the claims 1 to 11, or obtained by the process of any claims 12 to 14, for making various devices in the thoracic and cardiovascular surgical and/or medical field, such as a tissue patch, a papillary muscle for treating post-infarct pillar necrosis, a vascular prothesis, a device for treating myocardial conditions which combines a) a system for monitoring, remodeling or limiting the ventricular distension through containment, b) a system improving the myocardial contractility, a contractile device for releasing an active agent, a biological glue, a scaffold for cellular therapy and tissue engineery in the thoracic and cardiovascular field.
